(19) **Europäisches Patentamt**

**European Patent Office**

**Office européen des brevets**

(11) Veröffentlichungsnummer: **0 308 883 B1**

(12) **EUROPÄISCHE PATENTSCHRIFT**

(45) Veröffentlichungstag der Patentschrift: **17.05.95**

(21) Anmeldenummer: **88115460.3**

(22) Anmeldetag: **21.09.88**

Die Akte enthält technische Angaben, die nach dem Eingang der Anmeldung eingereicht wurden und die nicht in dieser Patentschrift enthalten sind.

(51) Int. Cl.⁶: **C12N 15/00**, C12N 1/20

(54) **Verfahren zum Klonieren von DNA-Sequenzen beziehungsweise zur Herstellung von Genbanken, Klonierungsvektoren und diese enthaltende Mikroorganismenstämme.**

(30) Priorität: **21.09.87 HU 424587**

(43) Veröffentlichungstag der Anmeldung:
**29.03.89 Patentblatt 89/13**

(45) Bekanntmachung des Hinweises auf die Patenterteilung:
**17.05.95 Patentblatt 95/20**

(84) Benannte Vertragsstaaten:
**AT BE CH DE ES FR GB IT LI NL SE**

(56) Entgegenhaltungen:
**US-A- 4 753 880**

**MANIATIS et al.: "Molecular cloning: A laboratory manual", 1. Edition, 1982, Cold Spring Harbor, New York, US; S. 13-15, 133-134, 269-274**

**GENE, Band 17, 1982, Seiten 27-44, Elsevier Biomedical Press; S. BRENNER et al.: "Phasmids: hybrids between ColE1 plasmids and E. coli bacteriophage lambda"**

(73) Patentinhaber: **BIOGAL GYOGYSZERGYAR Pallagi ut 13 H-4042 Debrecen (HU)**

(72) Erfinder: **Kiss, György Botond Dr. Kárász u. 16 H-6720 Szeged (HU)**
Erfinder: **Vincze, Eva Dr. Jankovich u.11 H-6726 Szeged (HU)**
Erfinder: **Ott, István Dr. Benczur u. 2 H-1068 Budapest (HU)**
Erfinder: **Kiss, Péter Epito u. 13a H-6723 Szeged (HU)**
Erfinder: **Klupp, Tibor Dr. Zöldlomp u. 19 H-1025 Budapest (HU)**
Erfinder: **Molnár, István Kossuth L. u. 32 H-1121 Budapest (HU)**
Erfinder: **Szeleczky, Zoltán Dr. Kolozsvár u. 7 H-1182 Budapest (HU)**

NATURE, Band 286, Nr. 5772, Juli 1980, Seiten 527-529, Basingstoke, GB; J.E.SUAREZ et al.: "DNA cloning in Streptomyces: a bifunctional replicon comprisingp BR322 inserted into a Streptomyces phage"

NATURE, Band 327, Nr. 6122, 11. - 17. Juni 1987, Seiten 532-535, Basingstoke, Hampshire, GB; W.R. JACOBS, Jr. et al.: "Introduction of foreign DNA intomycobacteria using a shuttle phasmid"

GENE, Band 40, 1985, Seiten 259-266, Elsevier Science Publishers; H.R. RACKWITZ et al.: "Analysis of cosmids using linearization by phage lambda terminase"

Maniatis et al.: "Molecular cloning: A laboratory manual", 1.Edition, 1982, Cold Spring Harbor, New York, US, S.295-299

Erfinder: **Ambrus, Gábor Dr.**
**Csalán u. 45/b**
**H-1025 Budapest (HU)**
Erfinder: **Moravcsik, Imre**
**Mester u. 38**
**H-1095 Budapest (HU)**

(74) Vertreter: **Beszédes, Stephan G., Dr.**
**Patentanwalt**
**Postfach 11 68**
**D-85201 Dachau (DE)**

**Beschreibung**

Die Erfindung betrifft ein Verfahren zum Klonieren von DNA-Sequenzen beziehungsweise zur Herstellung von Genbanken, Klonierungsvektoren und diese enthaltende Mikroorganismenstämme.

Es ist bekannt, daß unter Verwendung gentechnologischer Verfahren Gene oder gentragende DNA-Fragmente isoliert, in unterschiedliche lebende Zellen eingeschleust und die in ihnen enthaltenen genetischen Informationen ausgedrückt (exprimiert) werden können. Die neue Biotechnologie ist deshalb erfolgreich, weil

A) die Desoxyribonucleinsäure mit Restriktionsendonucleasen innerhalb der jeweiligen Nucleotidsequenz geschnitten werden kann,

B) die DNA-Fragmente mit biologischen und chemischen Verfahren kovalent miteinander gekoppelt werden können,

C) in prokaryontischen und eukaryontischen Systemen funktionierende DNA-Moleküle, sogenannte DNA-klonierende Vektoren, gefunden wurden, die in der Wirtszelle replikativ vermehrt werden und in die fremde DNA-Abschnitte eingebaut werden können, und

D) die rekombinante DNA in prokaryontische oder eukaryontische Zellen eingebracht werden kann, und die die fremde genetische Information tragenden Zellen selektiert werden können.

Das Klonieren eines Gens besteht darin, daß das durch Synthese, mittels reverser Transkriptase oder Restriktionsendonuklease hergestellte DNA-Fragment in eine Vektor-DNA eingebaut wird, die zur Replikation in einer Wirtszelle geeignet ist, und dann diese Rekombinante durch Transformation in eine Zelle eingeschleust wird. Bei der Herstellung von Genbanken werden Bakterienstämme isoliert, deren Gesamtheit alle Gene einer jeweiligen Zelle enthält, das heißt ihr vollständiges Genom. Eine Stammsammlung, die den gesamten Genbestand eines Bakteriums enthält, kann theoretisch erhalten werden, indem aus 5000 Basenpaaren bestehende Fragmente kloniert und 400 Transformanten isoliert werden.

Beim Klonieren vorher ausgewählter Gene und der Anlage von Genbanken ist der verwendete DNA-klonierende Vektor von ausschlaggebender Bedeutung. Es ist wichtig, daß der die fremde genetische Information tragende DNA-Vektor mit großer Wahrscheinlichkeit in die zu transformierende Zelle gelangt. Der Erfolg von Klonierungsversuchen wird wesentlich davon beeinflußt, in welchem Mengenanteil die das betreffende Gen tragenden DNA-Fragmente in den zur Transformation verwendeten, unterschiedliche DNA-Fragmente enthaltenden Gemisch vorliegen. Es ist klar, daß die Transformationsfrequenz für ein bestimmtes Gen um so größer ist, je mehr von den das gewünschte Fragment tragenden rekombinanten DNA-Vektoren bei der Transformation in die lebende Zelle gelangen.

Nach dem Schrifttum werden als Vektor-DNA Plasmide, Phagen beziehungsweise Virus-DNA, Cosmide und Phasmide verwendet.

Die Plasmide sind extrachromosomale genetische Elemente, sich vom Chromosom unabhängig replizierende, ringförmige DNA-s. Das erste als Vektor verwendbare Plasmid hatte das Zeichen pSC101. Später wurden noch zahlreiche weitere beschrieben, zum Beispiel ColE1, pBR322, pBR328, pMB9, PIJ702 oder PIJ41.

Der sich in E. coli vermehrende lambda-Phage wurde eingehend untersucht, gegenwärtig werden zum Klonieren lambda-Phagenmutanten verwendet. Die DNA des lambda-Phagen vom Wildtyp hat eine Größe von 49 kb. Nach der Infektion der Bakterienzelle kann sich der Phage im lysogenen oder im lytischen Zyklus vermehren. Wird der Phage als klonierender Vektor verwendet, dann muß seine Replikation im lytischen Zyklus erfolgen, wobei sich nach der Infektion die kohäsiven Enden (cos-Enden, sticky ends) der lambda-DNA miteinander verbinden und eine Replikation der zweisträngigen, ringförmigen DNA erfolgt. Der Phage enthält etwa 50 Gene, zu seinem Wachstum und der Bildung von Plaques sind jedoch nur 25 erforderlich. Die nicht-essentiellen DNA-Abschnitte können herausgeschnitten und durch fremde DNA ersetzt werden. Die rekombinante lambda-DNA verhält sich wie lambda-DNA. Gegenüber dem Plasmid hat der Phagenvektor den Vorteil, daß er in vitro zu lambda-Phagen umgebildet und nach in vitro erfolgendem Verpacken mit großer Frequenz in E. coli transduziert werden kann. ("Verpacken" von Phagen: die Phagen-DNA wird in einem Phagenköpfe, Phagenschwänze und Enzyme enthaltenden Gemisch inkubiert, wobei sich der Phage selbst "komplettiert". Vgl. auch: Assemblierung und Morphogenese, der Zusammenbau der Viruskomponenten zum fertigen Virion.). Die ersten Phagenvektoren waren lambda-gt WES, NM 641, NM 762 und die Charon-Serie (3, 21a usw.).

Die Cosmide enthalten einen aus einem Plasmid stammenden Abschnitt und die kohäsiven Enden des lambda-Phagen in Form einer doppelsträngigen DNA (cos-Ort). Der nahe am cos-Ort befindliche kurze Abschnitt wird in vitro und in vivo von dem Verpackungssystem erkannt. In gleicher Orientierung zwei cos-Orte enthaltende DNA, deren cos-Sequenzen durch einen Abschnitt der Länge von etwa 35 bis 53 kb getrennt sind, können in vitro verpackt und in E. coli transduziert werden. Innerhalb der Bakterie liegt das Cosmid in Ringform vor, gelangt jedoch nicht in einen lytischen Zyklus, weil es nicht sämtliche essentiellen lambda-Gene enthält. Gleichzeitig können jedoch, ähnlich wie bei Plasmiden als klonierenden Vektoren, die positiven Transformanten durch phänotypische Selektion, zum Beispiel auf der Basis ihrer Resistenz gegen Antibiotika, abgetrennt werden. Cosmidvektoren sind zum Beispiel pJC74, pJC720 oder MUA-3 und pJB8. (Schrifttum: Genetic Engineering, Ed. Robert Williamson; Dahl et al., Seiten 49 bis 69, 1981, Academic Press, London).

Neuerdings werden als klonierende Vektoren auch Phasmide eingesetzt. Das Phasmid ist ein als Phage isolierbarer, DNA-klonierender Vektor, der außer den kohäsive Enden am Einbauort der zu klonierenden DNA einen E. coli Replikationspunkt und ein gegen Antibioticum resistent machendes Gen enthält. Nach Einbau des zu klonierenden Fragmentes wird dieser Abschnitt abgestoßen, und das Hybridmolekül verhält sich im folgenden nur wie ein rekombinanter Phage, ist von diesem nicht zu unterscheiden (Karn, J. et al.: Proc. Natl. Acad. Sci., USA 77, 5172 bis 5176, 1980).

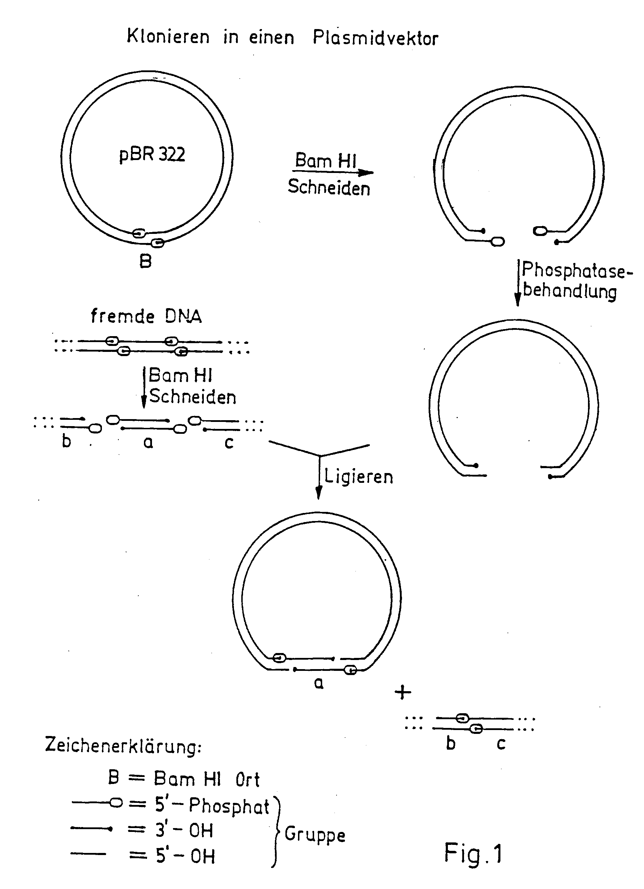

Es is bekannt, daß das zu klonierende DNA-Fragment in unterschiedlicher Gestalt in die Vektor-DNA eingebaut werden kann. Wenn die Vektor-DNA und die das zu klonierende Gen enthaltende Desoxyribonucleinsäure mit der gleichen Restriktionsendonuclease geschnitten werden, dann werden über sogenannte klebrige Enden verfügende Fragmente, die mit DNA-Ligase miteinander ligiert werden können, erhalten. Die fremde DNA kann in entsprechender Orientierung in den Vektor eingebaut werden, es können aber natürlich auch die klebrigen Enden der fremden DNA-Moleküle untereinander, die klebrigen Enden der Vektor-DNA untereinander und miteinander in Verbindung treten, wodurch eine außerordentliche Vielzahl neuer DNA-Moleküle entsteht. Wenn sich die beiden Enden des Vektors miteinander verbinden, entsteht wieder der ursprüngliche Vektor, und dieser macht auch bei optimalem Molverhältnis ein Mehrfaches der gewünschten Rekombinanten aus. Das ist nachteilig, und deshalb wird bei Plasmidvektoren die als kovalent ringförmig geschlossene Form (im folgenden ccc-Form) isolierte DNA mit einer Restriktionsendonuclease gespalten, die erhaltene lineare DNA mit alkalischer Phosphatase behandelt und dann das mit dem entsprechenden Enzym behandelte, zu klonierende Fragment eingebaut. Auf diese Weise kann verhindert werden, daß sich die Vektor-DNA beim Ligieren erneut zum Ring schließt, denn nach der Behandlung mit Phosphatase trennen sich die Phosphatgruppen von den 5′-Enden der DNA ab, und an den entstehenden Hydroxylgruppen kann beim Ligieren die kovalente chemische Bindung nicht entstehen. Die Einzelheiten des allgemein gültigen Verfahrens sind in Fig. 1 dargestellt.

Aus der Fig. 1 ist ersichtlich, daß nach dem Ligieren mehrere Arten neuer DNA-Moleküle entstehen können, aber nur das abgebildete ringförmige rekombinante Molekül lebensfähig ist.

Die kovalente chemische Bindung zwischen den freien 5′-Enden des mit Phosphatase behandelten Vektormoleküls und den freien 3′-Enden des Inserts wird nach der Transformation von der Zelle - durch Phosphorylieren und Ligieren in vivo - ausgebildet. Die in beiden Strängen Einzelstrangbrüche (nicks) enthaltenden rekombinanten Moleküle werden in vitro von den Wasserstoffbrücken zwischen den komplementären DNA-Strängen in Form eines Doppelstranges zusammengehalten. Anzahl und Art der unterschiedlichen rekombinanten Moleküle hängen von den Ligierungsbedingungen, von der Anzahl sämtlicher in das System eingebrachter Moleküle und von dem Molverhältnis des Vektors bzw. des fremden DNA-Fragmentes ab.

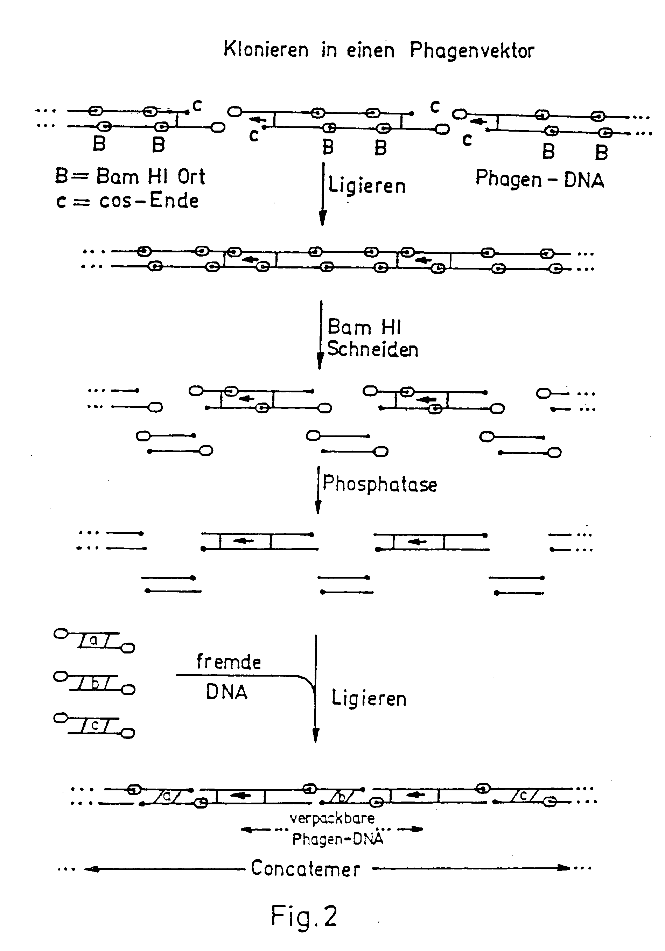

In Fig. 2 sind die bisher angewandten Klonierungsschritte für die Verwendung eines Phagenvektors schematisch gezeigt. Die als Phage isolierten, klebrige Enden enthaltenden, linearen DNA-Stücke werden ligiert. Dabei treten die Phagen über ihre klebrigen Enden mit sich selbst oder untereinander in kovalente Verbindung. Anschließend wird mit dem entsprechenden Enzym die keine essentiellen Gene enthaltende Mittelsequenz herausgeschnitten, und nach einer Phosphatesebehandlung wird der nunmehr an seinen Enden Hydroxylgruppen enthaltende Vektor mit dem an den Enden ausschliesslich Phosphatgruppen enthaltenden fremden DNA-Fragment ligiert. Aus Fig. 2 ist ersichtlich, daß bei unter geeigneten Bedingungen vorgenommener Ligierung infolge des Zusammenschlusses der klebrigen Enden eine sogenannte concatemere Form entsteht. Im Concatemer sind außer den die fremde DNA enthaltenden Abschnitten auch den Phagenvektor tragende Abschnitte vorhanden. Für den Fachmann ist offensichtlich, daß bei der Ligierung eine Vielzahl von in ihrer Zusammensetzung unterschiedlichen DNA-Molekülen entsteht, die jedoch keine lebensfähigen Phagen ergeben. Die Ligationsprodukte werden dann in einem Verpackungsgemisch in Phagen eingebaut und mit diesen die geeignete Wirtszelle transduziert.

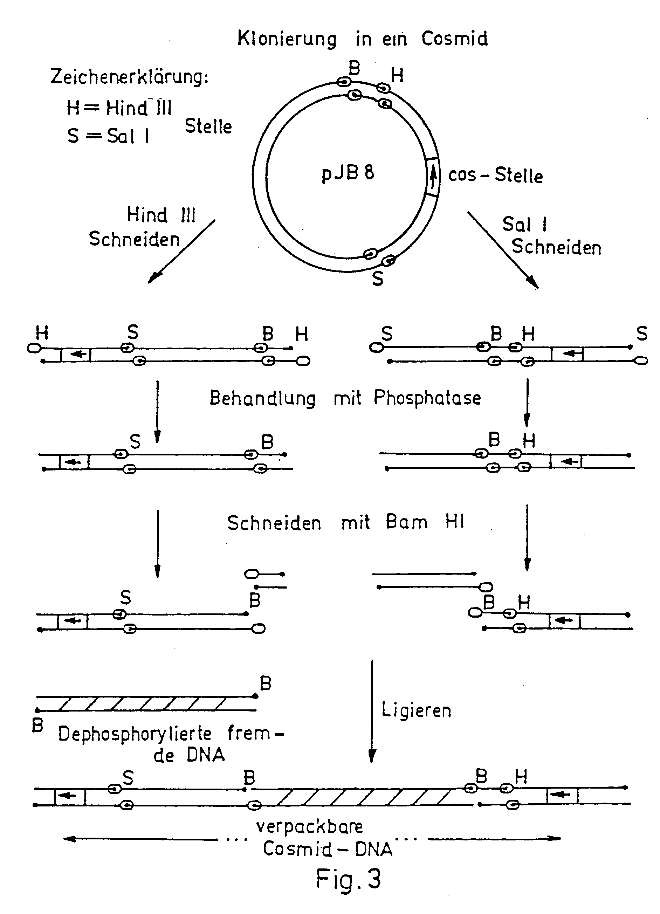

Von Ish-Horowitz und Burke (Nucleic Acid Research 9, 2989 bis 2998, 1981) wurde ein auf Phagenvektoren basierendes, zum Klonieren geeignetes System beschrieben, bei welchem ein Cosmid, das eine cos-Stelle enthält, in einem Reaktionsgefäß an der einen Seite der cos-Stelle mit einer Restriktionsendonuclease geschnitten wird, und eine weitere Menge des Moleküls in einem zweiten Reaktionsgefäß an der anderen Seite der cos-Stelle an einer anderen Restriktionsstelle geschnitten wird. Die beiden Ansätze werden vereinigt, die linearen Produkte werden mit alkalische Phosphatase behandelt und dann mit einer dritten Restriktionsendonuclease verdaut. Aus Fig. 3 ist ersichtlich, daß die vorher mit Phosphatase behandelte fremde DNA-Sequenz an mehreren Stellen eingebaut werden kann. Natürlich kann nur ein einziges Produkt in vitro verpackt (assembliert, komplettiert) werden.

Der Erfindung liegt die Aufgabe zugrunde, ein Verfahren zum Klonieren von DNA-Sequenzen beziehungsweise zur Herstellung von Genbanken mit besserer Ausbeute an den gewünschten Produkten bei Isolierbarkeit der rekombinanten Endprodukte sowohl als Phage als auch als Plasmid und besserer Selektivität, bei welchem lineare monomere DNA in höheren Anteilen in die Phagen eingebaut wird, diese eine erhöhte Assemblierbarkeit beziehungsweise Verpackbarkeit aufweisen und die das fremde DNA-Fragment enthaltenden Rekombinanten einfacher und effektiver abgetrennt werden können sowie durch welches beim Ligieren weniger unterschiedliche Produkte erhalten werden können, neue Klonierungsvektoren, mit welchen das Verfahren zum Klonieren von DNA-Sequenzen beziehungsweise zur Herstellung von Genbanken besonders vorteilhaft durchgeführt werden kann und diese Klonierungsvektoren enthaltende Mikroorganismenstämme zu schaffen.

Das Obige wurde überraschenderweise durch die Erfindung erreicht.

Erfindungsgemäß wird ein Verfahren zum Klonieren von DNA-Sequenzen beziehungsweise zur Herstellung von Genbanken bereitgestellt, wobei ein linearer Vektor verwendet wird, welcher zunächst mit Restriktionsendonuklease behandelt wird, und dem anschließend mit Phosphatase die Phosphatgruppen sowohl von den freien cos 5'-Enden als auch von den durch die Behandlung mit Restriktionsendonuklease erzeugten Restriktions-5'-Enden entfernt werden, zu diesen zur Ligation untereinander unfähigen Fragmenten werden dann die das zu klonierende Gen enthaltenden, an ihren Enden Phosphatgruppen aufweisenden DNA-Fragmente, zugegeben und das Gemisch mit Ligase ligiert, und die erhaltene monomere DNA in vitro verpackt und dann die Phagen vermehrt.

Im erfindungsgemäßen Verfahren werden anders ausgedrückt am Ende rekombinante lambda-Phagen in vitro verpackt und die Phagen vervielfältigt.

Nach einer vorteilhaften speziellen Ausführungsform des erfindungsgemäßen Verfahrens wird das Klonieren mit dem Phasmid pGY97 als DNA-klonierendem Vektor durchgeführt.

Nach einer weiteren vorteilhaften speziellen Ausführungsform des erfindungsgemäßen Verfahrens wird das Klonieren mit dem Phasmid pGYOKI9 als DNA-klonierendem Vektor durchgeführt.

Nach einer noch weiteren vorteilhaften speziellen Ausführungsform des erfindungsgemäßen Verfahrens wird das Klonieren mit den Phasmid pGYOKI10 als DNA-klonierendem Vektor durchgeführt.

Das erfindungsgemäße Klonierungsverfahren hat unter anderen folgende Vorteile Dadurch, daß die Bildung von Concatemeren durch die Behandlung mit Phosphatase verhindert wird, wird die Verpackbarkeit der Phagen in vitro erhöht. Aus den (gegebenenfalls rekombinanten, das heißt fremde DNA enthaltenden) Phasmiden (pGY97) und den erfindungsgemäßen Insertionsphasmiden (pGYOKI9 und pGYOKI10) können lineare Monomere hergestellt werden, wenn die Phasmide als Phagen-DNA isoliert werden. Es ist klar, daß das erfindungsgemäße Verfahren ebenso mit bekannten Phagenvektoren wie solchen, welche erst in der Zukunft eingeführt werden, durchführbar ist, es ist universell. Das Endprodukt kann im Gegensatz zu den bekannten Phagenvektoren sowohl als Phage als auch als Plasmid isoliert werden.

Die bei der Verwendung der Phasmide (wie pGY97) und neuen Phasmide (wie pGYOKI9 und pGYOKI10) erhaltenen rekombinanten Endprodukte können sowohl als Phage wie auch als Plasmid isoliert werden.

Dieser letztere Vorteil kann dadurch entstehen, daß die durch Verpacken in vitro gebildeten Rekombinanten in E. coli-Zellen, die den defekten Phagen mit der Mutation CIts 587 (E. coli K-1400) beberbergen, eingeschleust werden. Dadurch wird die Bildung reifer Phagen bei zum Beispiel 28°C durch das vom CI-Gen bestimmte Repressoreiweiß gehemmt. Bei 42°C jedoch wird das Repressoreiweiß inaktiviert und reife Phagen entstehen. Daher kommt es, daß aus den die rekombinanten Moleküle enthaltenden Zellen bei 42°C infolge der Funktion des lytischen Zyklus Phagen bzw. Plaques entstehen, während sich aus den gleichen Zellen bei 28°C unter selektiven Bedingungen, zum Beispiel auf Chloramphenicol oder Ampicillin enthaltenden Nährböden, Plasmide enthaltende Bakterienkolonien entwickeln.

Ein weiterer Vorteil des erfindungsgemäßen Klonierungsverfahrens besteht darin, daß - im Gegensatz zu den Phagenvektoren - die DNA-klonierenden Moleküle zur Selektion geeignet sind; sie enthalten einen Replikationspunkt und gegen Antibiotica resistent machende Gene, die in E. coli und/oder Streptomyces ausgedrückt werden.

DNA-Klonierungsvektoren vom Typ Phasmid, vorteilhaft verwendbar im obigen erfindungsgemäßen Klonierungsverfahren, werden hergestellt, indem die für die Phagenvermehrung unentbehrlichen Gene des Phagen lambda tragende Fragmente, ferner eine Replikation als Plasmid in E. coli-Zellen ermöglichende, eine Antibiotioumresistenz bestimmende DNA-Fragmente und schließlich das Überleben als Plasmid in einem anderen Mikroorganismus gewährleistende, in diesem Mikroorganismus selektierbare DNA-Fragmente - wobei die drei Fragmente zusammen die für die Phagenvermehrung erforderliche Größe haben - miteinander ligiert werden und die sowohl als Phage als auch als Plasmid isolierbare, zur Insertionsklonierung fremder DNA-Sequenzen geeignete, nach der Insertion in E. coli zur Replikation als Phage und als Plasmid befähigte, in dem anderen Mikroorganismus als Plasmid überlebende Phasmid-DNA isoliert wird.

Beispielweise wird beim Verfahren zur Herstellung der Phasmide pGYOKI9 und pGYOKI10 von dem rekombinanten Plasmid pGYOKI1/2 und dem Lambdaphagenvektor EMBL4 ausgegangen.

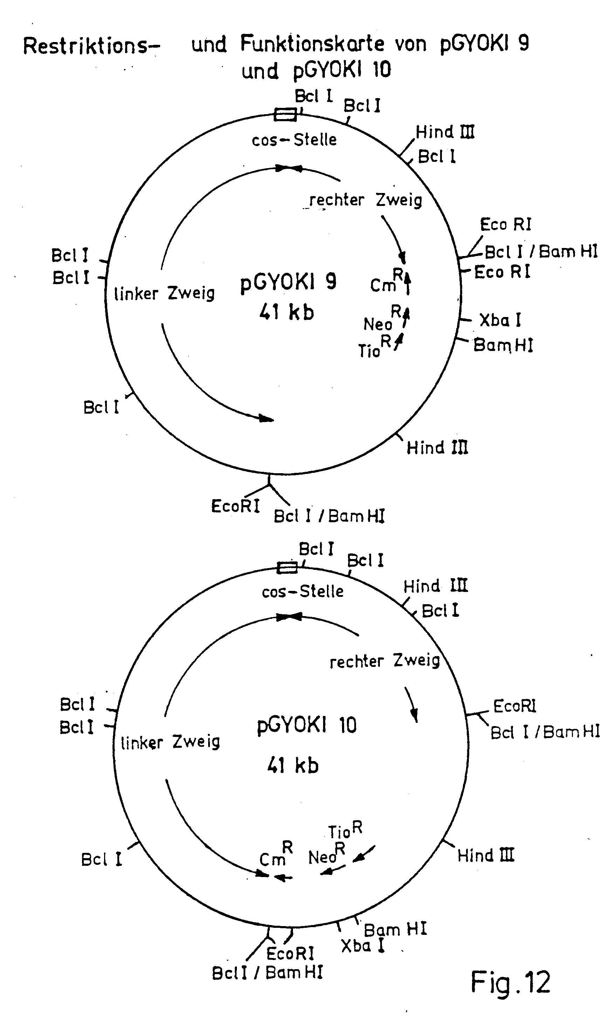

Weiterhin sind Gegenstand der Erfindung die Phasmide pGYOKI9 und pGYOKI10, welche die aus Fig. 12 ersichtlichen Restriktions- und Funktionskarten, je ein Chloramphenicol-, Neomycin- und Thiostrepton-Resistenzgen und eine Größe von 41 kb aufweisen. Sie sind nach dem obigen Verfahren zur Herstellung von DNA-Klonierungsvektoren vom Typ Phasmid erhältlich.

Außerdem sind Gegenstand der Erfindung der Mikroorganismusstamm Escherichia coli pGYOKI9 (NCAIM B/P/1011), enthaltend das obige Phasmid pGYOKI9 und der Mikroorganismusstamm Escherichia coli pGYOKI10 (NCAIM B/P/1012), enthaltend das obige Phasmid pGYOKI10.

Im erfindungsgemäßen Klonierungsverfahren können wie bereits gesagt Insertionsphasmide, wie pGY97, pGYOKI9 und pGYOKI10, verwendet werden.

Die Eigenschaften DNA-klonierender Vektoren sind in der folgenden Tabelle gezeigt.

Tabelle

| Vektor | Plasmid | Cosmid | Phage | Phasmid |
|---|---|---|---|---|
| Größe in kb | 8 bis 15 | 5 bis 20 | 45 | 45 |
| E. coli-Replikation | pl | pl | Phage | pl/Phage |
| Isolation | ccc | ccc | Phage | ccc/Phage |
| Klonierung | i | i | i/r | r |
| Größe von i in kb | 0 bis 30 | 20 bis 45 | 9 bis 22 | 9 bis 22 |
| Endprodukt | pl | pl | Phage | Phage |

i = Insertion
r = Substitution
pl = Plasmid
kb = Kilobase

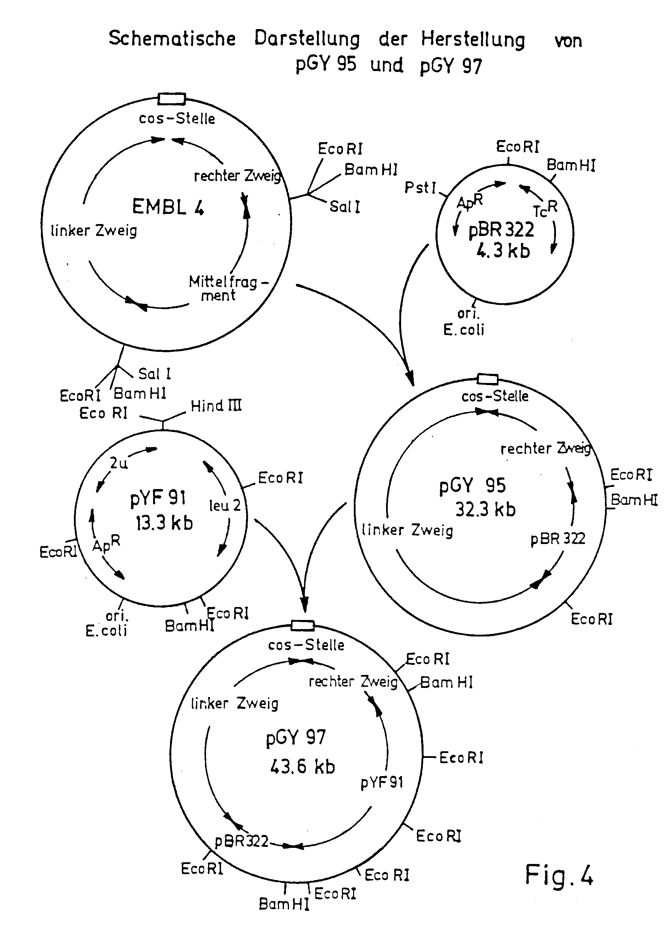

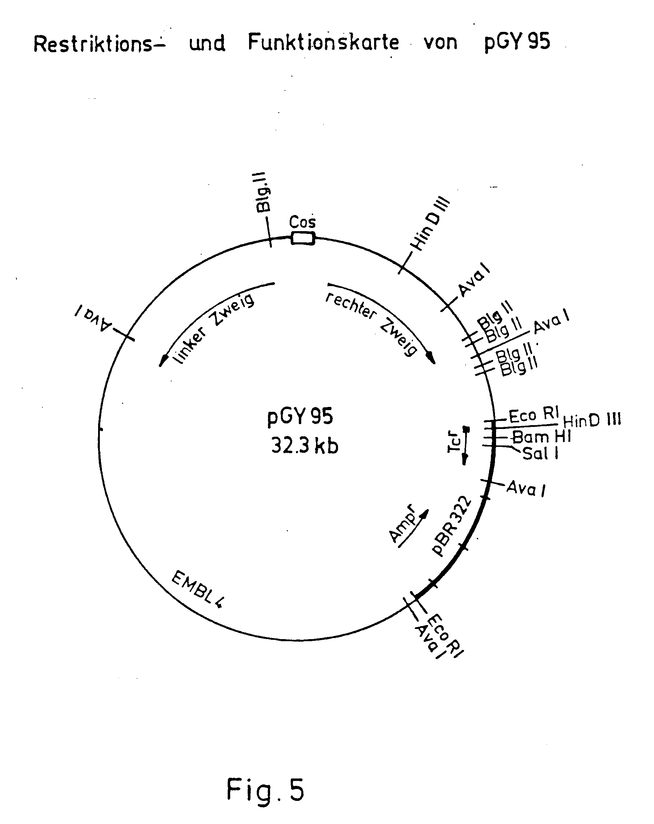

Erfindungsgemäß wird ein als Phage auch selbständig lebensfähiger DNA-Vektor vom Typ Phasmid hergestellt. Dabei wird beispielsweise vom sogenannten Transphamid pGY95 ausgegangen. Die Herstellung dieses Transphamides erfolgt zum Beispiel in der Weise, daß aus einem aus E. coli isolierten Plasmid, zum Beispiel dem Plasmid pBR322 (Bolivar, F. et al., Gene, 2, [1977], 95 bis 113), und einem lambda-Phagenvektor, zum Beispiel der DNA EMBL4 (Frischauf, A.M. und Mitarbeiter, J. Mol. Biol., 170 [1983], 827 bis 842), durch Verdauung mit Restriktionsendonuclease und Ligierung mit T4-DNA-Ligase der 32,3 kb große, ringförmige DNA-Vektor pGY95 hergestellt wird. Dieser Vektor wird als DNA-Vektor vom Transphamidtyp bezeichnet. Die einzelnen Schritte seiner Herstellung sind aus Fig. 4 ersichtlich. Das Transphamid enthält eine cos-Stelle, ein gegen Ampicillin resistent machendes Gen, einen E.-coli-Replikationspunkt und ein gegen Tetracyclin resistent machendes Gen, das bei einer BamHI-Insertion inaktiviert wird, bei einer partiellen EcoRI-Insertion jedoch intakt bleibt. Das Transphamid enthält weiterhin alle lambda-Gene, die zum lytischen Wachstum erforderlich sind. Die Restriktions- und Funktionskarte des Transphamides pGY95 sind wie bereits gesagt im einzelnen in Fig. 5 gezeigt. Zum Beispiel kann in das Transphamid pGY95 nach Öffnen mit BamHI das etwa 11,0 kb große Plasmid pYF91 (Storms, R. K. und Mitarbeiter, J. Bacteriol. 140 - [1979], 73 bis 82) eingebaut werden. Die einzelnen Schritte dieses Versuches sind aus Fig. 4 ersichtlich. Die entstandene ringförmige DNA pGY97 wurde auf Grund des Schrifttumes (Elledge, S. J. und Walker, G. C., J. Bacteriol., 162 [1985], 777 bis 783 als DNA-Vektor vom Phasmid-Typ bezeichnet. An Stelle von pYF91 können natürlich auch andere Plasmide oder DNA-Fragmente geeigneter Größe verwendet werden.

Das Phasmid pGY97 enthält außer den essentiellen $\lambda$-Phagengenen eine cos-Stelle, ein gegen Ampicillin resistent machendes Gen, einen E.-coli-Replikations punkt und ein gegen Tetracyclin resistent machendes Gen, das bei einer BamHI-Insertion inaktiviert wird, bei einer partiellen EcoRI-Insertion jedoch intakt bleibt. Die beim Klonieren gegen fremde DNA auswechselbare Sequenz befindet sich in dem Phasmid pGY97 zwischen zwei BamHI-Stellen.

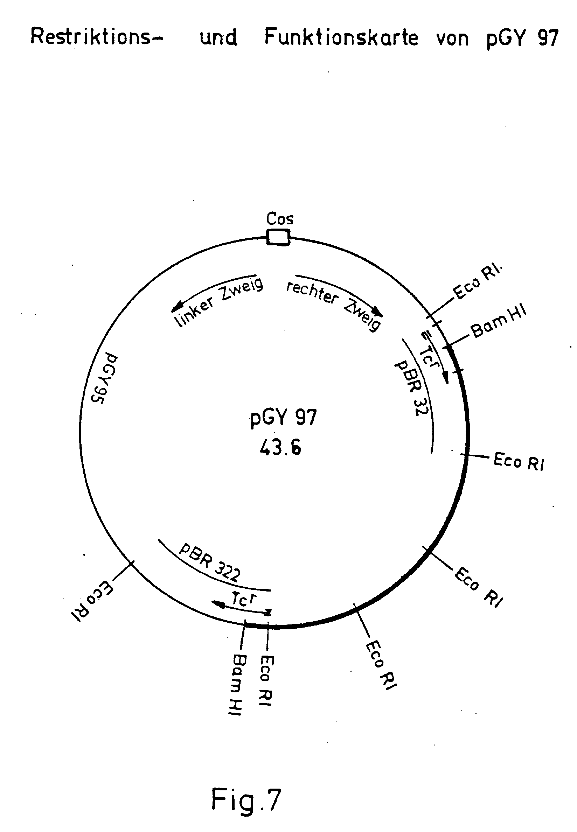

Die ausführliche Restriktions- und Funktionskarte von pGY97 ist in Fig. 7 abgebildet.

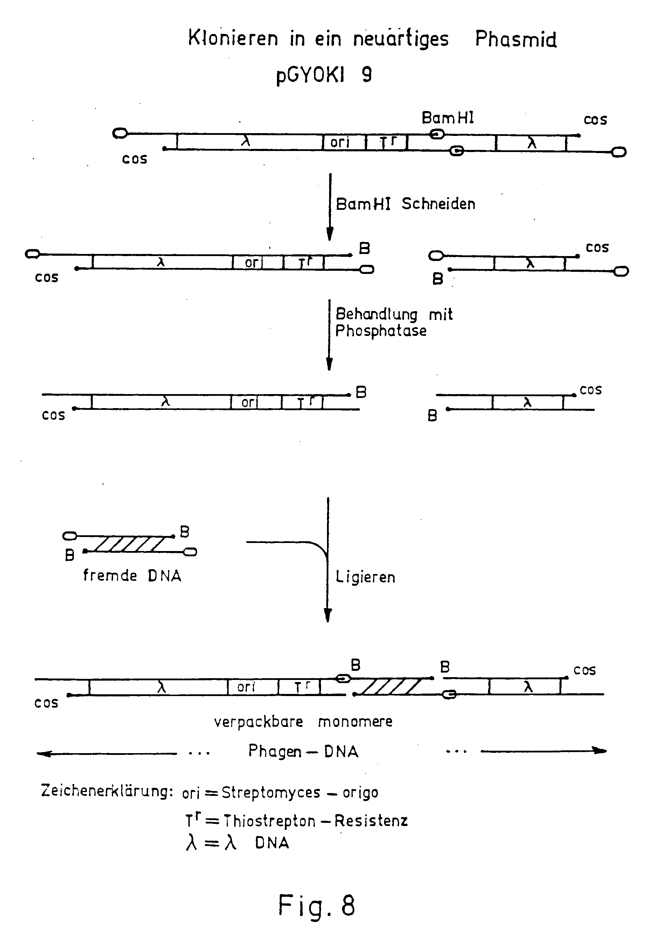

Beim Klonieren werden im wesentlichen die in Fig. 8 gezeigten Schritte vorgenommen. Das Phasmid wird als Phage isoliert und durch Schneiden mit BamHI wird das keine essentiellen Phagengene enthaltende Fragment entfernt. Dann werden die Phosphatgruppen sowohl von den kohäsiven lambda-Enden als auch von den nach der Restriktionsspaltung erhaltenen klebrigen BamHI-Enden mit Phosphatase entfernt. Zu dem Gemisch, das die drei an den Enden Hydroxylgruppen tragenden und deshalb zur Verbindung miteinander unfähigen Fragmente enthält, werden die das zu klonierende Gen enthaltenden, durch Schneiden mit BamHI (oder beispielsweise BclI, BglII, Sau3A I oder MboI) erhaltenen, an den Enden Phosphatgruppen aufweisenden Fragmente zugegeben und das Gemisch wird ligiert. Die cos-Enden treten, weil sie keine Phosphatgruppen aufweisen, nicht miteinander in Verbindung, das beißt Concatemere können nicht entstehen, das zu klonierende Fragment kann ausschließlich zwischen den beiden cos-Enden eingebaut werden. Eine Verbindung der zu klonierenden Sequenzen untereinander kann durch richtige Wahl des Verhältnisses zwischen Vektor und einzubauender Fragment-DNA praktisch vermieden werden.

Das die zu klonierende Sequenz enthaltende Phasmid wird anschließend in vitro verpackt. Wenn das Ligieren in Gegenwart von Polyäthylenglykol 6 000 vorgenommen wird (Pheiffer, B. H. und Zimmermann, B., Nucl. Acids Res., 22 [1983], 7 853), werden auf 1 µg Vektor-DNA bezogen auf $10^5$ bis $10^6$ rekombinante DNA enthaltende Zellen bzw. leere Flecken (Plaques) (siehe auch später) erhalten. Infolge der Behandlung mit Phosphatase enthalten alle nachweisbaren Phagen oder plasmidtragenden Zellen rekombinante DNA. Das bedeutet, daß die isolierbaren Phagen oder die rekombinante Phasmide tragenden Zellen theoretisch, wenn ein Insert von 11 kb zugrundegelegt wird, das komplette Genom einer beliebigen Bakterie mindestens 50-mal, das komplette Genom eines Pilzes 10-mal und theoretisch das komplette Genom eines Säugetiers enthalten.

Bei Durchführung der Klonierung mit den bisher bekannten Verfahren unter den bisher üblichen Bedingungen mit dem bisher üblichen Klonierungssystem ist die Anzahl der isolierbaren Rekombinanten um mindestens 1 Größenordnung kleiner.

Zur weiteren Veranschaulichung des erfindungsgemäßen Klonierungsverfahren wird das Klonieren des Genomes von Medicago sativa (Luzerne) in das Phasmid pGY97 auf die in den Beispielen ausführlich beschriebene Weise dargelegt. Aus Medicago sativa wird die Gesamt-DNA isoliert, mit Sau3A I geschnitten und die Fragmente der Größe von 9 bis 15 kb werden an einem Saccharose-Gradienten isoliert. Das Phasmid pGY97 wird als Phage isoliert, mit BamHI geschnitten und mit Phosphatase von den Phosphatgruppen befreit. Nach in Gegenwart von Polyäthylenglykol 6 000 vorgenommenem Ligieren werden in vitro Phagen hergestellt und in E. coli-Zellen transduziert und der Phagentiter wird bestimmt. Im Kontrollversuch waren keine Phagenplaques zu beobachten, im Klonierungsversuch konnten jedoch 5 . $10^5$ Phagen nachgewiesen werden. Das beweist, daß es gelungen war, eine Genbank anzulegen, die das Genom von Medicago sativa (Luzerne) mindestens 1-fach repräsentiert.

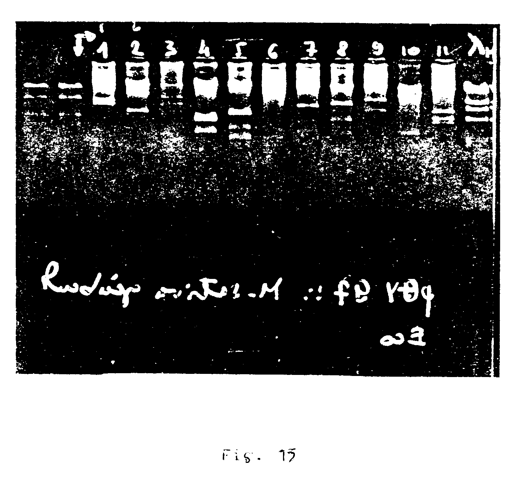

Zum Beweis des Obigen wurde aus 12 voneinander unabhängigen rekombinanten Klonen Plasmid-DNA isoliert, mit dem Restriktionsenzym EcoRI geschnitten, und die Restriktionsfragmente wurden mit Gelelektrophorese auf Agarose voneinander getrennt. Aus Fig. 15 ist ersichtlich, daß die einzelnen Klone aus Medicago sativa stammende EcoRI-Fragmente unterschiedlicher Größe enthalten.

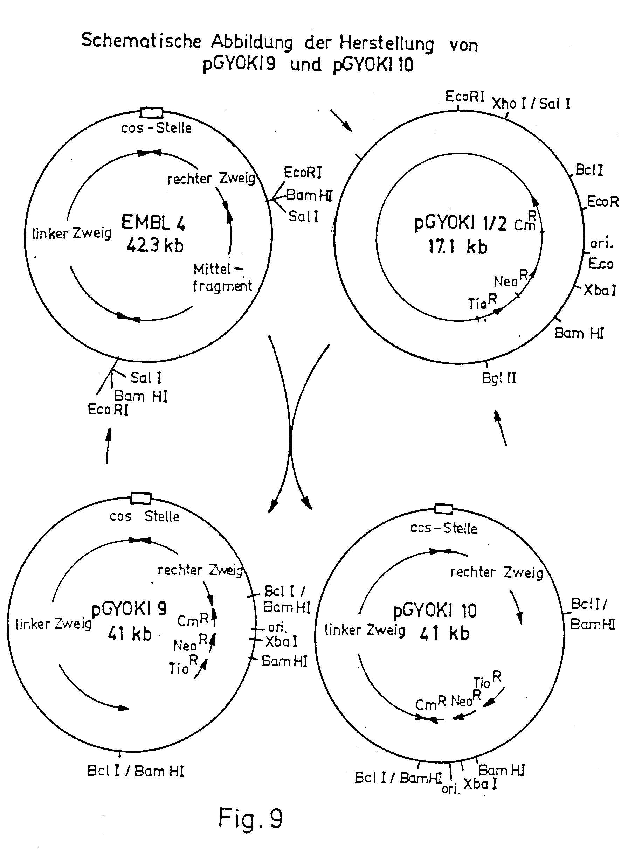

Zur Herstellung eines erfindungsgemäßen Phasmides, das eine auch in Streptomyces-Zellen stabil bleibende, selektierbare Antibioticumresistenz und einen Streptomyces-Replikationspunkt trägt, wird gemäß einer weiteren bevorzugten Ausführungsform der Erfindung an die Stelle des aus einem lambda-Phagenvektor, zum Beispiel aus der DNA EMBL4 (Frischauf, A. M. und Mitarbeiter, J. Mol. Biol. 170 [1983], 827 bis 842), mit BamHI herausgeschnittenen mittleren Fragmentes durch Ligieren mit T4-DNA-Ligase der aus dem Plasmid pGYOKI1/2 mit BclI herausgeschnittene, 12,8 kb große DNA-Abschnitt in beiden möglichen Orientierungen eingebaut, wobei die ringförmigen DNA-s pGYOKI9 und pGYOKI10 entstehen, die als erfindungsgemäßen DNA-Vektoren vom Phasmidtyp zu bezeichnen werden. Die einzelnen Schritte bei der Herstellung dieser neuen Vektoren pGYOKI9 und pGYOKI10 sind in Fig. 9 gezeigt.

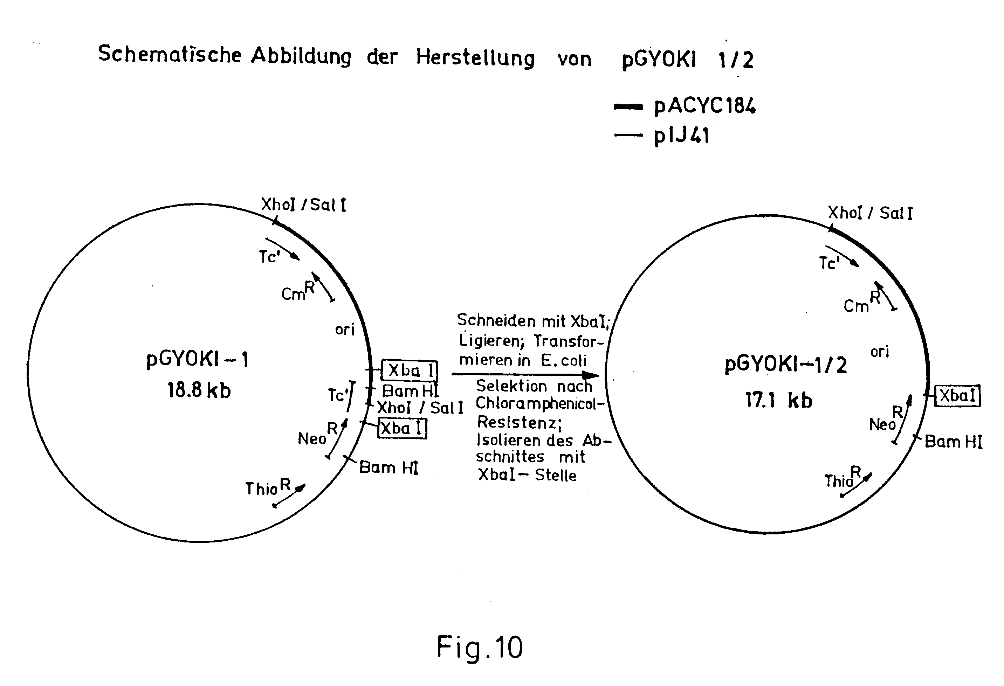

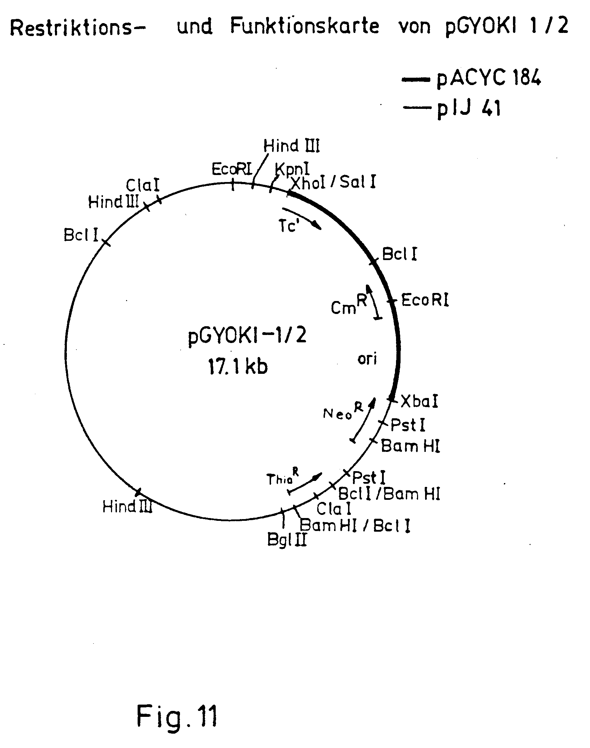

Die Herstellung des Plasmids pGYOKI1/2 ist in Fig. 10, seine Restriktions- und Funktionskarte in Fig. 11 gezeigt.

Die erfindungsgemäßen Phasmide pGYOKI9 und pGYOKI10 sind Insertionsphagenvektoren, sie sind jedoch auch als Plasmidvektoren funktionsfähig. Sie enthalten cos-Enden, in E. coli und davon unabhängig in Streptomyces funktionierende Replikstionspunkte, ein in E. coli ausdrückbares (exprimierbares), gegen Chloramphenicol resistent machendes Gen sowie Resistenzgene, die sich in Streptomyces äußern und gegen Thiostrepton sowie Neomycin resistent machen. Diese erfindungsgemäßen Phasmide enthalten eine einzige BamHI-Stelle; wenn in diese kloniert wird, verliert das gegen Neomycin resistent machende Gen seine Aktivität.

Die ausführliche Restriktions- und Funktionskarte von pGYOKI9 und pGYOKI10 ist wie bereits gesagt in Fig. 12 wiedergegeben.

Beim Klonieren werden im wesentlichen die in Fig. 8 bereits gezeigten und bei der Phasmidklonierung beschriebenen Schritte vorgenommen. Die erfindungsgemäßen Phasmide pGYOKI9 und pGYOKI10, welche die ersten Vertreter einer Gruppe von neuartigen Phasmiden sind, ähneln übrigens dem bereits beschriebenen Phasmid pGY97; da es sich jedoch bei den ersteren um Insertionsvektoren handelt, fällt beim Vorbereiten des Vektors nach dem Schneiden mit Restriktionsenzym kein mittleres Fragment an, wie dies bei den bisher bekannten Phasmiden der Fall ist. Bei den letzteren werden beim Ligieren nützliche, im übrigen klonierbare Sequenzen unklonierbar. Zur Vermeidung der Concatemerbildung kann auch bei diesen neuartigen Phasmiden die Behandlung der cos-Enden mit Phosphatase angewandt werden, wodurch die Häufigkeit der Klonierbarkeit um mindestens 1 Größenordnung erhöht wird.

Das hergestellte, die zu klonierende Sequenz tragende erfindungsgemäßen Phasmid wird in vitro verpackt. Wird das Ligieren in Gegenwart von Polyäthylenglykol 6 000 vorgenommen (Pheiffer, B. H. und Zimmermann, B., Nucl. Acids Res., 22 [1983], 7 853), dann werden mit einem Assemblierungsgemisch der Leistung von 1 bis 5 . $10^7$ Plaques pro 1 $\mu$g intakte lambda-DNA (siehe auch weiter unten) auf 1 $\mu$g Vektor-DNA bezogen $10^5$ bis $10^6$ Zellen, die rekombinante DNA enthalten, erhalten. Als Ergebnis der Behandlung mit Phosphatase enthalten alle nachweisbaren Phagen oder plasmidtragenden Zellen rekombinante DNA. Das bedeutet, daß die rekombinante DNA tragenden Zellen theoretisch, wenn mit einem Insert von 10 kb gerechnet wird, das gesamte Genom einer beliebigen Bakterie mindestens 50-mal, das eines Pilzes mindestens 5-mal und theoretisch mindestens $\frac{1}{4}$ des kompletten Genoms eines Säugetieres enthalten.

Bei Durchführung der Klonierung unter den bekannten Bedingungen mit einem der bekannten Kloniersysteme wäre die Anzahl der isolierbaren Rekombinanten um 1 Größenordnung geringer.

Zur weiteren Veranschaulichung des erfindungsgemäßen Verfahrens und der erfindungsgemäßen DNA-klonierenden Vektoren wird das Klonieren des Genomes von Streptomyces tendae in das erfindungsgemäße Phasmid pGYOKI9 auf die in den Beispielen ausführlich beschriebene Weise dargelegt. Aus Streptomyces tendae wird die Gesamt-DNA isoliert, mit Sau3A I geschnitten und die DNA-Fragmente einer Größe von 5 bis 9 kb werden an einem Saccharose-Gradienten isoliert. Das erfindungsgemaße Phasmid pGYOKI9 wird als Phage isoliert, mit BamHI geschnitten und mit Phosphatase von den Phosphatgruppen befreit. Nach in Gegenwart von Polyäthylenglykol 6 000 vorgenommenem Ligieren werden in vitro Phagen hergestellt, und mit E.-coli-Zellen wird durch Transduktion der Phagentiter bestimmt. In der Kontrolle sind keine Phagen bzw. Plaques nachweisbar, während im Klonierversuch die Gegenwart von 400 000 rekombinanten Phagen gezeigt werden kann. Das beweist, daß es gelungen ist, eine Genbank herzustellen, die das Genom von Streptomyces tendae mindestens 1-mal repräsentiert.

Die unter Verwendung des, Phasmids (pGY9J) und der erfindungsgemäßen Phasmide (pGYOKI9 und pGYOKI10) mittels des neuen Klonierungs verfahrens (Behandlung der cos-Enden mit Phosphatase isolierten DNA-s (oder Vektor-DNA-s) können, wie bereits erwähnt sowohl als Phage als auch als Plasmid isoliert werden. Das ist gegenüber den bekannten Vektoren, die entweder nur als Plasmid oder nur als Phage isoliert werden können, ein bedeutender Vorteil.

Es ist klar, daß die zum Klonieren verwendbaren DNA-Vektoren, Phasmid pGY97 und erfindungsgemäße Phasmide pGYOKI9 und pGYOKI10) nicht nur auf die oben kurz beschriebene und in den Beispielen näher erläuterte Weise hergestellt werden können. Derartige Vektoren können auch durch mit Restriktionsenzymen vorgenommenes Schneiden anderer Plasmide und Phagen-DNA-s und anschließendes Ligieren aus den dabei erhaltenen Produkten gewonnen werden, und ähnliche Ergebnisse stellen sich ein, wenn die auf diese Weise erhalteren Vektoren erfindungsgemäß kloniert werden. Die genannten klonierenden Vektoren können hergestellt und dann zum Beispiel mit den aus den folgenden Plasmiden, Phagen-DNA-s und Cosmiden hergestellten Vektor-DNA-s kloniert werden: Phagen, wie EMBL3 und Lambda 1059, Plasmide, wie pBR322, pYF91, pGYOKI3 und pGYOKI4, sowie Cosmide, wie pJB8.

In die erfindungsgemäßen DNA-Vektoren können auch andere Replikationspunkte, zum Beispiel die Replikation in Bacillus, Saccharomyces, Cephalosporium oder sonstigen Bakterien, in Pilzen, pflanzlichen oder tierischen Zellen ermöglichende Replikationspunkte eingebaut werden.

Es können auch gegen andere Antibiotica, zum Beispiel gegen Streptomycin, Tobramycin, Thiostrepton, Hygromycin oder Methotrexate oder gegen andere Verbindungen resistent machende Gene eingebaut werden. Auch diese Derivate fallen unter die Erfindung.

Die Mikroorganismen, welche die DNA-klonierenden Vektoren enthalten, werden bei der für den jeweiligen Mikroorganismus bevorzugten Temperatur an einem eine organische Kohlenstoffquelle, Stickstoffquelle und anorganische Salze enthaltenden Nährmedium gezüchtet. Im allgemeinen wird das Nährmedium durch für das Wachstum des Mikroorganismus notwendige Stoffe, wie Spurenelemente und Vitamine, ergänzt, obwohl diese Stoffe meistens in den Makrokomponenten des Nährmediums schon als Verunreinigungen enthalten sind. Die Mikroorganismen werden in belüfteter submerser Kultur in an sich bekannter Weise gegoren beziehungsweise fermentiert. Als Kohlenstoffquelle kommen zum Beispiel Glucose, Maltose und Stärke und als Stickstoffquelle anorganische Salze, Pepton, Hefeextrakt, Casein oder in der Gärungsindustrie allgemein bekannte sonstige Stoffe in Frage.

Die Phagen oder Plasmide können in an sich bekannter Weise isoliert werden. Das Fachschrifttum enthält dazu zahlreiche Hinweise (zum Beispiel Helms und Mitarbeiter, DNA, 4 [1985], 39). Auch die lineare DNA aus den Phagen kann in an sich bekannter Weise isoliert werden. Einige Verfahren werden in den Beispielen erläutert.

Die Erfindung wird an Hand der Beispiele 5 bis 10 näher erläutert. Die Beispiele 1 bis 4 sind nicht Gegenstand der vorliegenden Erfindung. Sie zeigen jedoch grundsätzliche Verfahrensschritte, auf die in den Beispielen 5 bis 10 bezug genommen wird.

Die bereits oben genannten und in den Beispielen erscheinenden Mikroorganismenstämme sind an der Budapester Universität für Gartenbau und Lebensmittelindustrie in der Sammlung Landwirtschaftlicher und Industrieller Mikroorganismen (NCAIM), Budapest, Ungarn hinterlegt.

Die in den Beispielen verwendeten Nährböden und Lösungen

## TA-Nährboden :
============

10 g Trypton (Bacto)
0,1 g Hefeextrakt (Difco)
5 g Natriumchlorid

werden in 1 liter destilliertem Wasser gelöst, zu der Lösung werden 1 ml 0,1 m wässrige Magnesiumchloridlösung und 1 ml 0,1 m Calciumchloridlösung gegeben. Das Gemisch wird bei 121 °C 20 Minuten lang sterilisiert.

## TGE-Lösung :

0,23 ml 40 gew.-%-ige wäßrige Glucoselösung,
0,25 ml 1 m tris-HCl-Lösung (pH 8),
0,4 ml 0,15 m wässrige EDTA-Lösung (Reanal Budapest)
9,12 ml ionenfreies Wasser.

## NSE-Lösung :

13,9 ml destilliertes Wasser
1,33 ml 3 m Natronlauge
0,8 ml 25 gew.-%-ige wäßrige Natriumlaurylsulfatlösung
4 ml 0,25 m wäßrige EDTA-Lösung

TE-Lösung :
=========

250 ml 0,01 m tris-HCl-Lösung (pH 7,5)
1 ml 0,25 m wäßrige EDTA-Lösung

SM-Lösung :
=========

20 ml 1,0 m tris-HCl-Lösung (pH 7,5)
5,8 g NaCl
2,0 g Magnesiumsulfat
20 g Gelatine
Dest. Wasser zum Auffüllen auf 1 Liter.
Die Lösung wird bei 121° C 20 Minuten lang sterilisiert.

NTM-Puffer :
==========

10 ml 1,0 m NaCl-Lösung
20 ml 1,0 m tris-HCl-Lösung (pH 7,5)
10 ml 1,0m MgCl$_2$-Lösung
960 ml ionenfreies Wasser.

EC-Puffer :
=========

340 $\mu$l 3 m wässrige NaCl-Lösung
2000 $\mu$l 1 m tris-HCl-Lösung (pH 7,5)
120 ml 1 m MgCl$_2$-Lösung
8,4 $\mu$l Mercaptoäthanol
7430 $\mu$l dest. Wasser.

L-Lösung :
=========

1,25 ml 1 m tris-HCl -Lösung (pH 8)
0,25 ml 1 m wäßrige MgCl$_2$-Lösung
0,5 ml 1 m Dithiothreit-Lösung (BRL, Mary
land, USA) 30,3 mg Adenosintriphosphat
3 ml ionenfreies Wasser.

B-Puffer :
==========

1000 $\mu$l 3 m wäßr. NaCl
120 $\mu$l 1 m tris-HCl-Lösung (pH 7,5)
120 $\mu$l MgCl$_2$
8,4 $\mu$l Mercaptoäthanol
8650 $\mu$l dest. Wasser.

D-Puffer:
=========

50 ml 80 gew.-%-ige Saccharose-Lösung
0,01 g Bromphenolblau
0,4 ml 1 m tris-HCl-Lösung (pH 7,5)
4 ml dest. Wasser.


BeM-Nährboden:
==============

1,0 Gew.-% Dextrin
0,1 Gew.-% Hefeextrakt
0,2 Gew.-% hydrolysiertes Casein
0,1 Gew.-% Fleischextrakt (LabLemco)
0,001 Gew.-% Kobaltchlorid
2,0 Gew.-% Agar (Bacto)
Auffüllen mit Leitungswasser


BI-Nährboden. :
===============

| | |
|---|---|
| Hefeextrakt | 0,3 Gew.-% |
| Pepton | 0,5 Gew.-% |
| Malzextrakt | 0,3 Gew.-% |
| Glucose (getrennt sterilisiert) | 1,0 Gew.-% |
| Magnesiumchlorid | 0,1 Gew.-% |
| Saccharose | 17,0 Gew.-% |


Lysozym-Lösung :
================

100 ml 10,3 gew.-%-ige Saccharose-Lösung
1 ml 2,5 gew.-%-ige Kaliumsulfatlösung
1 ml 0,5 gew.-%-ige Kaliumdihydrogenphosphatlösung
0,1 ml 2,5 m $MgCl_2$ -Lösung
1 ml 0,25 m $CaCl_2$ -Lösung
2,5 ml 1 m tris-HCl-Lösung (pH 8,0)
4 ml 50 gew.-%-ige Glucoselösung
100 mg Lysozym (Reanal, Budapest).


NESP-Puffer :
=============

128 ml destilliertes Wasser
5 ml 3 m NaCl-Lösung
30 ml 0,250 m EDTA-Lösung 30 ml 10 gew.-%-ige Natriumlauryl
sulfat-Lösung

3 ml Proteinaselösung (20 mg/ml, Merck)

## GVI/b-Nährboden:
================

0,1 Gew.-% Calciumcarbonat
0,1 Gew.-% Kaliumnitrat
0,1 Gew.-% Kaliumdihydrogenphosphat
0,5 Gew.-% Ammoniumnitrat
0,05 Gew.-% Natriumchlorid
0,05 Gew.-% Magnesiumsulfat
0,1 Gew.-% Asparagin
2,0 Gew.-% wasserlösliche Stärke
1,9 Gew.-% Agar (Bacto)

Der pH-Wert des mit Wasser aufgefüllten Nährmediums wird vor dem Sterilisieren auf 7,3 eingestellt, dann wird bei 121 °C 30 Minuten lang sterilisiert.

Beispiel 1

Herstellung des Transphamids pGY95

12,5 µl der gemäß dem unten folgenden Abschnitt I b der Ausgangsmaterialherstellung gereinigten DNA-Lösung des Plasmids pBR322 werden zu 12,5 µl EC-Puffer gegeben. Zu dem Reaktionsgemisch werden 5 Einheiten Restriktionsenzym EcoRI gegeben (BRL, Maryland, USA), and das Gemisch wird bei 37 °C eine Stunde lang stehengelassen.

12,5 µl der gemäß dem unten folgenden Abschnitt II b der Ausgangsmaterialherstellung gereinigten DNA von EMBL4 werden zu 12,5 µl EC-Puffer gegeben und das Gemisch dann mit 5 Einheiten Restriktionsenzym EcoRI versetzt. Auch dieses Gemisch wird bei 37 °C eine Stunde lang inkubiert.

Das Restriktionsenzym EcoRI schneidet beide DNA in der Sequenz GAATTC. An beiden DNA entstehen nach der Spaltung aus vier Basenpaaren bestehende überhängende Enden (klebrige Enden, sticky ends), die einander komplementär sind und daher in jeder Kombination miteinander in Verbindung treten können.

Beide Reaktionsgemische werden 10 Minuten lang auf 68 °C gehalten und dann miteinander vereinigt. Zu der die linearen DNA-Moleküle enthaltenden Lösung werden 12,5 µl L-Lösung gegeben. Das Gemisch wird, damit sich die linearen DNA-Moleküle zusammenschliessen, durch eine Einheit T4-DNA-Ligase (BRL, Maryland, USA) ergänzt und dann bei 15 °C 16 Stunden lang inkubiert. Nun erscheint im Reaktionsgemisch das Transphamid pGY95. Der schematische Ablauf der Herstellung des Transphamids pGY95 ist in Fig. 4 gezeigt. Fig. 5 zeigt die ausführliche Restriktions- und Funktionskarte des Plasmids.

Die in diesem Beispiel verwendeten Ausgangsmaterialien sind wie folgt beschrieben hergestellt worden.

I) Herstellung des Plasmids pBR322

a) Züchtung von Escherichia coli pBR322 (NCAIM B/P/359; hinterlegt am 1. Juni 1986)

Der Stamm E. coli pBR322 (NCAIM B/P/359) wird bei -20 °C aufrechterhalten. Er wird bei 28 °C bis zur Erreichung der Phase des stationären Wachstums auf TA-Nährboden gezüchtet. 3 ml der Kultur werden mit 0,225 ml Dimethylsulfoxyd vermischt, plötzlich auf -70 °C abgekühlt und bei dieser Temperatur gelagert.

Zur Züchtung der Zellen werden 100 ml TA-Nährboden in einem Erlenmeyerkolben von 500 ml Volumen sterilisiert. Der sterile Nährboden wird mit 0,1 ml der im eingefrorenen Zustand gelagerten Kultur beimpft. Der Ansatz wird bei 28 °C 16 Stunden lang auf einer Schüttelmaschine der Frequenz von 200 $min^{-1}$ kultiviert.

b) Isolierung des Plasmids pBR322

Die gemäss Punkt a) hergestellte Kultur wird in einer Zentrifuge Sorvall RC5 bei 4 °C mit einer Drehzahl von 8000 $min^{-1}$ zentrifugiert. Die Zellen werden in 10 ml TGE-Lösung suspendiert. Zu der Suspension werden 20 ml NSE-Lösung gegeben. Das sich klärende Lysat wird bei Raumtemperatur 5 Minuten lang stehengelassen, dann mit 15 ml 5 m Natriumacetatlösung (pH 4,8) versetzt und 5 Minuten lang bei 0°C stehengelassen. Die entstandene Suspension wird bei 0 °C in einem Rotor der Drehzahl 10 000 min$^{-1}$ 5 Minuten lang zentrifugiert. Das Zentrifugat wird abgetrennt und mit 0,54 Volumenteilen Isopropanol der Temperatur von -20 °C versetzt. Der Ansatz wird bei -20 °C 30 Minuten lang stehengelassen. Bei 0°C wird durch Zentrifugieren mit einer Drehzahl von 10 000 min$^{-1}$ die ausgeschiedene DNA abgetrennt. Der Niederschlag wird mit 10 ml 70 gew.-%-igem Äthanol gewaschen und dann im Exsiccator getrocknet. Das Produkt wird in 7 ml TE-Lösung aufgelöst. In den erhaltenen 7 ml Lösung werden 7,5 g Cäsiumchlorid (SERVA) gelöst, dann werden 0,5 ml einer pro ml 10 mg Äthidiumbromid enthaltenden Lösung zugegeben. Dann wird der Ansatz in einem Rotor T865.1 bei 15 °C 48 Stunden lang mit einer Drehzahl von 39 000 min$^{-1}$ in einer Ultrazentrifuge Sorvall OTD-50B zentrifugiert. Der fluoreszierende untere Streifen wird mit einer Injektionsnadel entfernt, das Äthidiumbromid wird mit Isopropanol dreimal extrahiert. Die erhaltene, Plasmid-DNA enthaltende Lösung wird gegen auf das 1000fache verdünnte sterile TE-Lösung dialysiert; auf diese Weise wird die reine DNA des Plasmids pBR322 erhalten. Die DNA-Konzentration wird durch Messung der optischen Durchlässigkeit bei 260 nm berechnet (eine OD260-Einheit entspricht 50 $\mu$g/ml DNA.)

Die ausführliche Restriktions- und Funktionskarte des Plasmids ist in Fig. 4 dargestellt.

II) Isolierung des Phagen EMBL4

a) Reinigung des Phagen EMBL4

Die Stammlösung des Phagen wird bei 4 °C in einer mit Chloroform bereiteten Suspension gelagert (Frischauf et al., J. Mol. Biol., 170, 827-842 /1983/). Zu 4 ml der Phagensuspension EMBL4, die den auf TA-Nährmedium wachsenden Stamm E. coli NM526 (NCAIM B /P/1006; hinterlegt am 4. Juni 1987) lysiert, wird 0,1 ml Chloroform gegeben, der Ansatz wird gründlich geschüttelt und bei 4°C gelagert.

Die Phagenstammlösung wird so weit verdünnt, dass voneinander getrennte Plaques entstehen; damit werden Zellen von E. coli NM526 (NCAIM B/P/1006) infiziert. Ein alleinstehender Plaque wird abgenommen und in 1 ml SM-Puffer suspendiert. Die Suspension wird bei 0 °C 4-6 Stunden lang schwach geschüttelt. Aus 0,1 ml der Phagensuspension wird ein Plattenlysat bereitet. Die Platten werden bei 37 °C 8-12 Stunden lang inkubiert. Zu der völlig klargewordenen Platte werden 6 ml SM-Puffer gegeben, unter schwachem Schütteln wird bei 0 °C inkubiert.

5 ml dieses Lysats werden zu einer sich auf 500 ml TA-Nährmedium exponentiell teilenden (OD600 = 0,3) Bakterienkultur gegeben, die dann bei 37 °C auf der Schüttelmaschine inkubiert wird. Der OD600-Wert der Kultur wird alle 30 Minuten gemessen. Ist das Minimum erreicht, so wird die Kultur auf Raumtemperatur gekühlt und mit 5 ml Chloroform versetzt. Der Ansatz wird geschüttelt, bis zu einer Endkonzentration von 1 $\mu$g/ml mit RNaz (SIGMA) versetzt und weitere 30 Minuten lang inkubiert.

Zu 500 ml Kultur werden 29,2 g kristallines Kochsalz gegeben, das Salz wird durch Rühren aufgelöst und die Lösung eine Stunde lang stehengelassen. Dann werden die Zellenbruchstücke durch Zentrifugieren entfernt (Drehzahl des Rotors 11 ooo min$^{-1}$). Zentrifugiert wird bei 4 °C 10 Minuten lang. Der klare Überstand wird gesammelt und bis zu einer Endkonzentration von 10 Gew.-% mit Polyäthylenglycol 6000 (PEG; FLUKA) versetzt. Nachdem sich das PEG gelöst hat, wird der Ansatz bei 0 °C eine Stunde lang stehengelassen und dann der Niederschlag durch bei 4 °C 10 Minuten lang vorgenommenes Zentrifugieren (Drehzahl des Rotors 11 000 min$^{-1}$) abgetrennt. Der Überstand wird entfernt, der Niederschlag wird in 8 ml SM-Lösung vorsichtig suspendiert und mit dem gleichen Volumen Chloroform extrahiert. Die beiden Phasen werden durch Zentrifugieren voneinander getrennt, und zu der wässrigen Phase werden pro ml 0,5 g Cäsiumchlorid (SERVA) gegeben. Nachdem alles in Lösung gegangen ist, wird die Phagensuspension vorsichtig an einem Cäsiumchlorid-Konzentrationsgradienten geschichtet.

Der Gradient wird auf folgende Weise bereitet:

| Lösung | Dichte (g/ml) | CsCl (g) | SM (ml) | Refraktionsindex (n) |
|---|---|---|---|---|
| a | 1,45 | 60 | 85 | 1.3768 |
| b | 1,50 | 67 | 82 | 1.3815 |
| c | 1,70 | 95 | 75 | 1.3990 |

Vor der Lösung c werden 3 ml in ein Kunststoffzentrifugenröhrchen SW28 (Sorvall) pipettiert, diese werden vorsichtig mit 3 ml Lösung b überschichtet, und auf diese gelangen 4 ml Lösung a. Dieser Gradient wird nun mit den 20 ml Phagensuspension überschichtet. In einer Ultrazentrifuge Sorvall OTD-50B wird das Röhrchen in einem Rotor SW28 mit einer Drehzahl von 22 000 min$^{-1}$ bei 4 °C 2 Stunden lang zentrifugiert. Der Phagenstreifen (ein opaler Streifen zwischen den Lösungen a und b) wird mit einer Injektionsnadel abgesaugt und mit so viel CsCl-Lösung b der Konzentration 1,5 g/ml versetzt, daß das Endvolumen 10 ml beträgt. Diese Lösung wird in ein Kunststoffzentrifugenröhrchen gegossen und in einem Rotor SW50.1 bei 4 °C und einer Drehzahl von 38 000 min$^{-1}$ mit der oben erwähnten Zentrifuge 24 Stunden lang zentrifugiert.

Der Phagenstreifen wird mit einer Injektionsnadel abgesammelt und gegen des 1000fache Volumen NTM-Lösung dialysiert.

b) Die Herstellung der Phagen-DNA EMBL4

Nach der Dialyse werden zu 1,0 ml Phagensuspension EDTA (500 mM, pH 8,0, bis zu einer Endkonzentration von 20 mM), Proteinase K (50 $\mu$g/ml) und Natriumlaurylsulfatlösung (SDS;, 20 Gew.-%, bis zu einer Endkonzentration von 0,5 Gew.-%) gegeben. Das Gemisch wird bei 37°C eine Stunde lang inkubiert.

Nach der Verdauung wird die Lösung vorsichtig mit einem Gemisch von Phenol und Chloroform im Volumverhältnis von 1 : 1 vermischt, dann werden die beiden Phasen durch Zentrifugieren (10 Minuten, 10 000 min$^{-1}$) voneinander getrennt. Die wäßrige Phase wird gegen das 1 000-fache Volumen TE-Puffer dialysiert. Die DNA-Konzentration wird auf die unter I) beschriebene Weise bestimmt.

Die Restriktions- und Funktionskarte des Phagen ist in Fig. 4 dargestellt.

Beispiel 2

Herstellung von E. coli pGY95 (NCAIM B/P/356; hinterlegt am 1. Juni 1986)

Mit 10 $\mu$l des gemäss Beispiel 1 hergestellten, das Transphamid pGY95 enthaltenden Reaktionsgemisches werden Zellen von E. coli K-1400 (NCAIM B/P/357; hinterlegt am 1. Juni 1986) transformiert.

Der Stamm E. coli K-1400 wird auf die bei der Ausgangsmaterialherstellung des Beispieles 1, Abschnitt I a) beschriebene Weise auf TA-Nährboden geimpft und kultiviert. Die pro ml 2.10$^8$ Zellen enthaltende Kultur wird bei 4 °C in einer Zentrifuge Sorvall RC5 mit einer Drehzahl von 5000 min$^{-1}$ 5 Minuten lang zentrifugiert. Das Abgesetzte wird in 1,2 ml 0,02 m wässrigem tris-HCl-Puffer (pH 7,5) suspendiert. Zu 0,5 ml dieser Bakteriensuspension werden 10 $\mu$l der gemäss Beispiel 1 hergestellten Transphamid-DNA-Lösung sowie 0,1 ml 0,15 m wässrige Calciumchloridlösung und 0,13 m wässrige Magnesiumchloridlösung gegeben. Das Gemisch wird bei 0 °C 60 Minuten lang stehengelassen. Dann wird die Suspension mit 5 ml TA-Nährboden versetzt und bei 28 °C 30 Minuten lang inkubiert. Dann werden die Zellen durch Zentrifugieren wir oben beschrieben abgetrennt.

Die isolierten transformierten Zellen werden auf dem festen Nährboden TAA, der 50 $\mu$g/$\mu$l Ampicillin enthält, vereinzelt. Der Nährboden TAA hat die gleiche Zusammensetzung wie der Nährboden TA, enthält jedoch zusätzlich 2,2 Gew.-% Agar (Bacto). Die Kulturen werden bei 28°C 24 Stunden lang inkubiert. Die ampicillin-resistenten Kolonien werden isoliert (Transformationsfrequenz: 10 000 Transformanten pro 1 $\mu$g DNA), wie bei der Ausgangsmaterialherstellung des Beispieles 1, Abschnitt I a) beschrieben gezüchtet und die Plasmid-DNA wird wie bei der Ausgangsmaterialherstellung des Beispieles 1, Abschnitt I b) beschrieben abgetrennt. Das abgetrennte Transphamid pGY95 wird mit Endonucleasen analysiert. Als solche werden EcoRI, BamHI und HindIII verwendet (BRL, Maryland, USA). Die enzymatische Behandlung wird gemäss Beispiel 1 vorgenommen mit dem Unterschied, dass beim Schneiden mit BamHI beziehungsweise HindIII B-Puffer verwendet wird, der je eine Einheit BamHI beziehungsweise HindIII enthält. Zu je 10 $\mu$l der behandelten Proben werden je 10 $\mu$l D-Puffer gegeben, und die DNA-Fragmente werden durch Elektrophorese an Agarosegel voneinander getrennt. Zur Herstellung des Agarosegels wird 1 g Agarose (SIGMA, St. Louis, USA) zu 80 ml TEA-Puffer gegeben und die Suspension 2 Minuten lang gekocht, dann auf 60 °C abgekühlt und zu einem horizontalen Gel vergossen. Zur Herstellung des TEA-Puffers werden in 800 ml destilliertem Wasser 48,4 g Tris, 3,7 g EDTA-dinatrium, 16,4 g Na-Acetat und 0,05 ml Äthidiumbromidlösung einer Konzentration von 10 mg/ml aufgelöst. Der pH-Wert wird mit konzentrierter Essigsäure auf 8,3 eingestellt und das Gemisch mit destilliertem Wasser auf 1000 ml aufgefüllt.

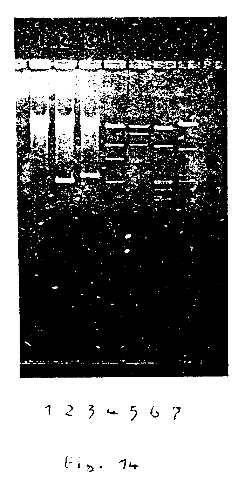

Die Lösungen, die die mit Restriktionsenzym behandelten pGY95-DNA-Fragmente enthalten, werden auf das Gel aufgetropft und in TEA-Puffer 4 Stunden lang einer Elektrophorese mit 40 V und 40 mA unterworfen. Die DNA-Fragmenten unterschiedlicher Länge entsprechenden DNA-Banden werden auf Grund ihrer Fluoreszenz unter UV-Licht identifiziert und dann photographiert. Das Ergebnis der Restriktionsanalyse ist in Fig. 14 dargestellt.

Die auf Grund dieser Ergebnisse konstruierte Restriktions-und Funktionskarte ist in Fig. 5 gezeigt.

Der das Transphamid enthaltende E. coli Stamm wurde unter der Bezeichnung E. coli pGY95 bei der Sammlung Landwirtschaftlicher und Industrieller Mikroorganismen hinterlegt und erhielt die Nummer NCAIM B/P/356.

Beispiel 3

Isolierung des Transphamids pGY95 aus E. coli pGY95

Der Stamm E. coli pGY95 (NCAIM B/P/356) wird wie bei der Ausgangsmaterialherstellung des Beispieles 1, Abschnitt I a) beschrieben gezüchtet und das Transphamid wie bei der Ausgangsmaterialherstellung des Beispieles 1, Abschnitt I b) beschrieben isoliert.

Beispiel 4

Herstellung einer Genbank von Streptomyces tenebrarius AR-20 (NCAIM P/B/169) im Transphamid pGY 95

a) Vorbereitung und Ligieren des Vektors und der Insert-DNA

12,5 $\mu$l der gemäss Beispiel 3 gereinigten pGY95-Transphamid-DNA-Lösung werden zu 12,5 $\mu$l B-Puffer gegeben. Das Gemisch wird mit 5 Einheiten des Restriktionsenzyms BamHI versetzt (BRL, Maryland, USA) und dann eine Stunde lang bei 37 °C inkubiert. Dann wird das Gemisch mit Phosphatase behandelt. Dazu wird die mit BamHI geschnittene DNA mit 20 Einheiten alkalischer Phosphatase versetzt (Calf Intestinal, Boehringer Mannheim GmbH) und zuerst 15 Minuten bei 37 °C, dann 15 Minuten bei 56 °C inkubiert. Dann wird die Probe in dem gleichen Puffer bei 68 °C 60 Minuten lang mit 20 Einheiten bakterieller alkalischer Phosphatase behandelt (BRL, Maryland, USA). Nach der Phosphatasebehandlung wird auf die bei der unten folgenden Ausgangsmaterialherstellung beschriebene Weise Phenol zugesetzt, die DNA mit Äthanol ausgefällt und in 20 $\mu$l TE-Lösung aufgenommen.

Die Effektivität des Schneidens mit BamHI und der Phosphatasebehandlung wird durch Ligieren (mit nicht phosphatasebehandelten BamHI-Fragmenten) und erneutes Schneiden (BamHI) überprüft. Wenn die BamHI-Behandlung in Ordnung war, müssen beim erneuten Schneiden die Ausgangsfragmente zurückerhalten werden. Der mit Phospatase behandelte Vektor kann mit sich selbst kein Ligierungsprodukt bilden.

125 $\mu$l der auf die bei der unten folgenden Ausgangsmaterialherstellung beschriebene Weise gereinigten Gesamt-DNA-Lösung von Streptomyces tenebrarius AR-20 (NCAIM P/B/169) werden zu 125 $\mu$l B-Puffer gegeben; dann wird der Ansatz mit einer Einheit des Restriktionsenzyms Sau3AI (BRL, Maryland, USA) versetzt und bei 37 °C 10 Minuten lang inkubiert. Der Zusatz von Phenol und das Ausfällen der DNA mit Äthanol erfolgen auf die bei der unten folgenden Ausgangsmaterialherstellung beschriebene Weise. Die DNA wird in 500 $\mu$l TE-Lösung aufgenommen.

Durch die Behandlung mit Sau3AI wird die DNA partiell gespalten, und es entstehen Fragmente unterschiedlicher Grösse. Da die in den Vektor optimal einbaubaren Fragmente eine Grösse von 9-15 kb haben, wird die partiell gespaltene DNA zur Isolierung dieser Fragmente an einem Saccharosegradienten aufgetrennt.

Beim Zentrifugieren trennen sich die Fragmente im Gradienten ihrer Grösse nach, auf diese Weise können die Fragmente geeigneter Grösse isoliert werden.

Im einzelnen verläuft die Fraktionierung nach Grösse wie folgt. In einem SW41-Zentrifugenröhrchen wird ein kontinuierlicher Saccharose-Dichtegradient von 10-40 Gew.-% Konzentration hergestellt. Mit TE-Puffer wird eine 10 gew.-%-ige und eine 40 gew.-%-ige Saccaroselösung bereitet (der TE-Puffer wurde vorher mit 1 m Natriumchlorid versetzt). Je 6 ml der beiden Lösungen werden in den Gradientenmischapparat, ins Zentrifugenröhrchen gegeben. Ganz oben auf kommt die Probe (500 $\mu$l). Dann wird bei 4 °C und einer Rotordrehzahl von 32 000 min $^{-1}$ mit einer Ultrazentrifuge des Typs Sorvall OTD-50B 16 Stunden lang zentrifugiert. Das Zentrifugat wird in Fraktionen zu je 200 $\mu$l geteilt, die Grösse der in den Fraktionen befindlichen DNA wird durch Gelelektrophorese auf Agarose in der im Beispiel 2 beschriebenen Weise bestimmt. Die entsprechenden Fraktionen werden vereinigt, die DNA mit Äthanol ausgefällt und dann in 10 $\mu$l TE-Lösung aufgelöst.

Das Restriktionsenzym BamHI schneidet die DNA bei der Sequenz GGATCC, das Enzym Sau3AI schneidet die Sequenz GATC. Es entstehen an beiden Basenpaaren überstehende Enden (sticky ends) aus 4 Basenpaaren, die einander komplementär sind und deshalb in jeder Kombination miteinander in Verbin-dung treten können.

10 $\mu$l des mit BamHI geschnittenen Transphamids pGY95 werden mit 10 $\mu$l der mit Sau3AI partiell gespaltenen DNA-Lösung von Streptomyces tenebrarius AR-20 (NCAIM P/B/169) wie folgt ligiert: Zu den 20 $\mu$l Reaktionsgemisch werden 5 $\mu$l L-Puffer und 5 Einheiten T4-DNA-Ligase gegeben. Die Arbeitsweise ist die gleiche wie in Beispiel 1.

Nach dem Ligieren wird das Gemisch in zwei Hälften geteilt. Die eine Probe (12,5 $\mu$l ) wird mit TE-Lösung auf 48 $\mu$l verdünnt und dann mit 2 $\mu$l Terminaselösung bie 37 °C 30 Minuten lang behandelt. Das Enzym Terminase wird gemäss Rackwitz u.a. (Gene, 40, 259-266 /1985/) hergestellt.

b) Phagenerzeugung in vitro

Während des Ligierens erscheinen im Reaktionsgemisch die concatemeren beziehungsweise in dem mit Terminase behandelten Reaktionsgemisch die monomeren DNA-Moleküle, die zwischen zwei Vektormolekülen entsprechender Orientierung die geeignet grossen, das ganze Genom umfassenden DNA-Fragmente von Str. tenebrarius enthalten. Diese Moleküle werden unter Verwendung eines Assemblierungsgemisches in vitro zu reifen Phagen ausgebildet.

Unter Phagenreifung, dem Verpacken des Phagen in vitro wird hier verstanden, dass die im Reagenzglas befindliche DNA durch Zusatz geeigneter Lysate (SE beziehungsweise FTL; die eine Komponente enthält die Phagenköpfe und -schwänze, die andere Enzyme) und eines Puffers zu reifen Phagen mit einer Hülle auswächst. Das Assemblierungsgemisch ist ein Handelsprodukt der Bezeichnung DNA-Kit AO1-0215 und wird von der Firma Biotechnika Rt. (Szeged, Ungarn) hergestellt.

Zur 8 $\mu$l des Kit-Puffers werden 10 $\mu$l des wie oben beschrieben hergestellten Ligats und dann 2 $\mu$l auf Eis geschmolzenes SE-Lysat und 5 $\mu$l FTL-Lysat gegeben. Das Gemisch wird sofort umgerührt und 10 Minuten lang bei 0 °C, dann 60 Minuten lang bei 25 °C stehengelassen. Dann wird das Gemisch mit 975 $\mu$l SM-Puffer versetzt und pro 100 $\mu$l gegen E. coli NM526 (NCAIM B /P/1006) titriert. Die Züchtung des Wirtsorganismus und das Titrieren werden gemäss der Benutzungsanleitung zum DNA-Kit AO1-0215 vorgenommen, als Nährboden dient TAA-Medium. Nach 12stündigem Wachstum werden pro Platte 1500 Plaques gezählt. Bei der Kontrollklonierung (Ligieren ohen die DNA von Streptomyces tenebrarius AR-20 {NCAIM P/B/169}) zeigt sich kein einziger Plaque. Das bedeutet, daß eine Genbank hergestellt wurde, die das Genom von Streptomyces tenebrarius AR-20 (NCAIM P/B/169) in mehrfacher Ausfertigung enthält.

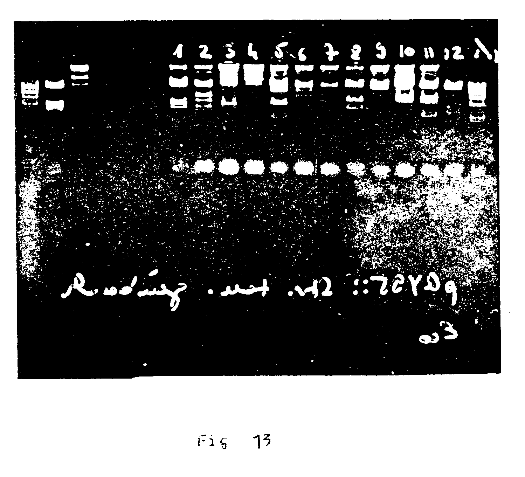

Ein weiterer Beweis für die Entstehung der Genbank besteht darin, dass bei einer Untersuchung von 12 unabhängigen Plaques jedes einzelne eine rekombinante DNA enthielt (s. Fig. 13). Mit von den 12 unabhängigen Plaques stammenden Phagen wurde E.coli K-1400 (NCAIM B/P 357) infiziert. Die Populationen werden bei 28°C wie bei der Ausgangsmaterialherstellung des Beispieles 1, Abschnitt la beschrieben gezüchtet, wobei sich die rekombinante DNA in Plasmidform vervielfältigt. Dann wird die Plasmid-DNA wie bei der Ausgangsmaterialherstellung des Beispieles 1, Abschnitt I b) beschrieben isoliert, gemäß Beispiel 1 mit EcoRI geschnitten, und die entstandenen Fragmente werden wie im Beispiel 2 beschrieben durch an Agarose vorgenommene Elektrophorese analysiert. Das Ergebnis der Gelelektrophorese ist in Fig. 13 dargestellt.

Die in diesem Beispiel als Ausgangsmaterial verwendete Gesamt-DNA-Lösung von Streptomyces tenebrarius AR-20 (NCAIM P/B/169 ist wie folgt beschrieben hergestellt worden.

a) Züchtung von Streptomyces tenebrarius AR-20 (NCAIM P/B/169; hinterlegt am 1. Juni 1986)

Der Stamm Streptomyces tenebrarius AR-20 NCAIM P/B/169 wird auf dem agarhaltigen Nährboden BeM kultiviert. Der mit Leitungswasser bereitete Nährboden wird von dem Sterilisieren auf pH 7,2 eingestellt und dann bei 121°C 30 Minuten lang sterilisiert.

Die Schrägagar werden mit den Sporen von Streptomyces tenebrarius AR-20 (NCAIM P/B/169) beimpft und dann bei 37°C 6 Tage lang inkubiert. Mit der gewachsenen Kultur werden 100 ml BI-Nährboden beimpft (der Nährboden wurde in einem 500 ml Erlenmeyerkolben bei 121°C 20 Minuten lang sterilisiert, vorher jedoch auf pH 7,0 eingestellt).

Die Kulturen werden bei 37 °C 24 Stunden lang auf einer Schüttelmaschine inkubiert, die auf einen Kreis von 2,6 cm Durchmesser minütlich 260 Drehungen ausführt. Mit je 1 ml der Kultur werden BI-Nährböden beimpft, die 1, 2 beziehungsweise 3 Gew.-% Glycin enthalten. Diese werden auf die beschriebene Weise 24 Stunden lang inkubiert. Die Kultur, auf der Mycele wachsen, wird auf Nährböden mit steigender Glycinkonzentration übergeimpft. Auf diesen wird so lange gezüchtet, bis der Mikroorganismus auf 5 Gew.-% Glycin enthaltendem Nährboden innerhalb 24 Stunden wächst.

b) Die Isolierung der Gesamt-DNA von Streptomyces tenebrarius AR-20 (NCAIM P/B/169)

Die gemäss a) hergestellte Kultur wird in einer Zentrifuge Sorvall RC5 bei 25 °C 10 Minuten lang zentrifugiert (Drehzahl des Rotors 8000 min $^{-1}$). Die Zellen werden in 200 ml 10 gew.-%-iger steriler Saccaroselösung suspendiert und dann erneut abzentrifugiert. Anschliessend werden die Mycele in 10 ml Lysozymlösung suspendiert, in dieser 60 Minuten lang bei 30 °C inkubiert und dann mit 4,6 ml 0,25 m EDTA-Lösung und mit 1,6 ml Pronase (Calbiochem, USA) einer Konzentration von 10 mg/ml versetzt. Nach 5 Minuten bei 30 °C wird der Ansatz mit 0,66 ml 25 gew.-%-igem Natriumlaurylsulfat versetzt und erneut bei 37°C 60 Minuten lang inkubiert.

Dann wird dem Ansatz das gleiche Volumen eines im Volumverhältnis 1:1 bereiteten Gemisches aus Phenol und Chloroform zugefügt, und nach vorsichtigem Rühren werden die beiden Phasen durch Zentrifugieren voneinander getrennt (10 Minuten mit 10 000 min$^{-1}$). Die wässrige Phase wird dreimal mit Äther extrahiert und dann mit dem 2,5fachen Volumen 96 gew.-%-igen Aethanols versetzt. Die ausgefallene DNA wird durch Zentrifugieren abgetrennt (Sorvall RC5, 5 Minuten mit 10 000 min$^{-1}$). Der klare Überstand wird entfernt, die DNA im Exsiccator getrocknet und dann in 7 ml TE-Lösung aufgenommen.

Die isolierte DNA wird wie bei der Ausgangsmaterialherstellung des Beispieles 1, Abschnitt I b) beschrieben durch in Gegenwart von Cäsiumchlorid und Äthidiumbromid vorgenommenes Ultrazentrifugieren gereinigt und dann dialysiert. Die reine Gesamt-DNA ist nach dem Zentrifugieren in einem einzigen fluoreszierenden Streifen enthalten.

Beispiel 5

a) Herstellung des rekombinanten Phasmids pGY97

12,5 μl der auf die bei der unten folgenden Ausgangsmaterialherstellung beschriebene Weise hergestellten gereinigten DNA-Lösung des Plasmids pYF91 werden zu 12,5 μl Puffer gegeben. Dieses Gemisch wird mit 5 Einheiten des Restriktionsenzyms BamHI versetzt (BRL, Maryland, USA) und dann eine Stunde lang bei 37°C inkubiert.

12,5 μl des gemäß Beispiel 3 erhaltenen gereinigten Transphamids pGY95 werden zu 12,5 μl Puffer gegeben, das Gemisch dann mit 5 Einheiten des Restriktionsenzyms BamHI versetzt und ebenfalls bei 37 °C eine Stunde lang stehengelassen. Das Enzym BamHI schneidet beide DNA in der Sequenz GGATCC. Dabei bilden sich aus vier Basenpaaren bestehende überstehende Enden (sticky ends) an beiden DNA; diese überstehenden Enden sind einander komplementär und können daher in beliebiger Kombination miteinander in Verbindung treten.

Die beiden Reaktionsgemische werden miteinander vermischt, dann wie bei der Ausgangsmaterialherstellung des Beispieles 4 beschrieben mit Phenol versetzt und die DNA mit Äthanol ausgefällt. Der DNA-Niederschlag wird in 50 μl TE-Puffer aufgelöst und die Lösung mit 12,5 μl L-Lösung versetzt. Zu dem Gemisch wird, damit die linearen DNA-Moleküle sich verbinden, eine Einheit T4-DNA-Ligase (BRL, Maryland, USA) gegeben und dann bei 15 °C 16 Stunden lang inkubiert. Im Reaktionsgemisch erscheint das rekombinante Phasmid pGY97, dessen Herstellung schematisch Fig. 4 darstellt, während die ausführliche Restriktions- und Funktionskarte des Phasmids aus Fig. 7 zu entnehmen ist.

b) Herstellung von E. coli pGY97 (NCAIM B/P/358; hinterlegt am 1. Juni 1986)

5 μl des wie unter a) beschrieben hergestellten Reaktionsgemisches, das das rekombinante Phasmid pGY97 enthält, werden zur in-vitro-Verpackung verwendet. Diese wird gemäss Beispiel 4b) vorgenommen. Mit den erhaltenen reifen Phagen, in denen das Phasmid pGY97 vorliegt,werden Zellen von Escherichia coli K-1400 (NCAIM B/P/357) infiziert. Nach der Infektion werden die das Phasmid pGY97 enthaltenden Zellen auf festem, pro ml 50 μg Ampicillin enthaltendem TAA-Nährboden vereinzelt. Die Kulturen werden bei 28 °C 24 Stunden lang inkubiert. Die ampicillin-resistenden Kolonien werden abgetrennt (Verpackungshäufigkeit in vitro 10 000 Transformanten/μg DNA), wie bei der Ausgangsmaterialherstellung des Beispieles 1, Abschnitt I a) beschrieben gezüchtet und die Phagen-DNA wird wie bei der Ausgangsmaterialherstellung des Beispieles 1, Abschnitt II b) beschrieben isoliert. Das isolierte Phasmid pGY97 wird wie im Beispiel 2 beschrieben durch Schneiden mit Endonucleasen analysiert. Als Restriktionsenzyme werden EcoRI, BamHI und HindIII verwendet (BRL, Maryland, USA). Zu je 10 μl der mit Enzym behandelten Proben werden je 10 μl D-Puffer gegeben, und die entstandenen DNA-Fragmente werden auf die im Beispiel 2 beschriebene Weise mittels Gelelektrophorese an Agarosegel getrennt.

Das Ergebnis der Restriktionsanalyse ist in Fig. 14 abgebildet. Fig. 7 zeigt die aus dem Ergebnis der Restriktionsanalyse abgeleitete Restriktions- und Funktionskarte.

Der das rekombinante Phasmid pGY97 enthaltende Stamm E. coli wurde als E. coli pGY97 bezeichnet und bei der Sammlung Landwirtschaftlicher und Industrieller Mikroorganismen unter der Nummer NCAIM B/P/358 hinterlegt.

c) Isolierung des rekombinanten Phasmids pGY97 als Phage aus E. coli pGY97 (NCAIM B/P/358)

Der Stamm E. coli pGY97 (NCAIM B/P/358) wird wie bei der Ausgangsmaterialherstellung des Beispieles 1, Abschnitt I a) beschrieben gelagert und gezüchtet, jedoch mit dem Unterschied, daß die Inkubationstemperatur 28°C beträgt. Zur Reinigung des als Phage anfallenden Phasmids pGY97 wird die Kultur bei 37°C gegen E. coli NM526 (NCAIM B/P/1006) titriert. Bei dieser Temperatur vermehr sich das Phasmid in Phagenform. Die aus E. coli NM526 (NCAIM B/P/1006) erhaltenen Phagen werden dann wie bei der Ausgangsmaterialherstellung des Beispieles 1, Abschnitt II a) beschrieben vermehrt, und die DNA wird wie bei der Ausgangsmaterialherstellung des Beispieles 1, Abschnitt II b) beschrieben isoliert.

Die in diesem Beispiel als Ausgangsmaterial verwendete gereinigte DNA-Lösung des Plasmids pYF91 ist wie folgt beschrieben erhalten worden.

Reinigung des Plasmids pYF91

Der Stamm E. coli pYF91 (NCAIM B/P/1007; hinterlegt am 4. Juni 1987) wird wie bei der Ausgangsmaterialherstellung des Beispieles 1, Abschnitt I a) beschrieben gelagert und kultiviert. Das Plasmid wird wie bei der Ausgangsmaterialherstellung des Beispieles 1, Abschnitt I b) beschrieben isoliert.

Die ausführliche Restriktions- und Funktionskarte des Plasmids ist in Fig. 4 dargestellt.

Beispiel 6

Herstellung der Genbank von Medicago sativa in dem Phasmid pGY97

12,5 $\mu$l der gemäß Beispiel 5, Abschnitt c) isolierten Phagen-DNA-Lösung des Plasmids pGY97 werden zu 12,5 $\mu$l B-Puffer gegeben. Dem Reaktionsgemisch werden 5 Einheiten des Restriktionsenzyms BamHI (BRL,Maryland, USA) zugesetzt. Dann wird das Gemisch bei 37 °C eine Stunde lang inkubiert und anschliessend wie in Beispiel 4a) beschrieben mit Phosphatase behandelt.

125 $\mu$l der wie bei der unten folgenden Ausgangsmaterialherstellung beschrieben erhaltenen gereinigten DNA von Medicago sativa werden zu 125 ml B-Puffer gegeben. Das Gemisch wird mit einer Einheit Restriktionsendonuclease Sau3AI (BRL, Maryland, USA) versetzt und dann bei 37 °C 10 Minuten lang inkubiert. Das Gemisch wird auf die bei der Ausgangsmaterialherstellung des Beispieles 4, Abschnitt b) beschriebene Weise mit Phenol versetzt, die DNA dann mit Äthanol ausgefällt und in 500 $\mu$l TE-Lösung aufgelöst.

Das Enzym Sau3AI spaltet die DNA partiell, es entstehen Fragmente unterschiedlicher Grösse. Da Fragmente der Grösse 5-15 kb optimal in den Vektor eingebaut werden können, wird zur Isolierung von Fragmenten dieser Grösse das enzymatisch behandelte Gemisch an einem Saccharose-Gradienten aufgetrennt. Der Gradient wird auf die im Beispiel 4a) beschriebene Weise zentrifugiert. Danach werden Fraktionen zu je 200 $\mu$l genommen, und durch Gelelektrophorese auf Agarose wird die Grösse der in jeder Fraktion vorhandenen DNA-Fragmente bestimmt. Die geeigneten Fraktionen (5-15 kb, vorzugsweise 9-15 kb) werden vereinigt, mit Äthanol ausgefällt und in 10 $\mu$l TE-Lösung aufgelöst.

Das Restriktionsenzym BamHI schneidet die DNA innerhalb der Sequenz GGATCC, das Enzym Sau3AI bei der Sequenz GATC. Nach der Spaltung entstehen an beiden DNA aus vier Basenpaaren bestehende kohäsive Enden, die einander komplementär sind und deshalb in beliebiger Kombination miteinader in Verbindung treten können.

Die partiell gespaltene DNA von Medicago sativa wird in das mit BamHI geöffnete und mit Phosphatase behandelte Phasmid pGY97 ligiert. Dazu werden je 1o $\mu$l der beiden Lösungen miteinander vermischt und mit 5 $\mu$l LP-Mischung versetzt. Zur Herstellung der LP-Mischung werden 1,25 ml m Tris-HCl-Lösung (pH 8,0) und 0,25 ml m wäßrige Magnesiumchloridlösung, 0,5 ml m Thiothreit-Lösung (BRL, Maryland, USA), 2,5 ml 30 gew.-%-iges Polyäthylenglykol 6000, 30,3 mg Adenosintriphosphat und 0,5 ml ionenfreies Wasser miteinander vermischt.

Zum Koppeln der linearen DNA-Moleküle wird dem Gemisch eine Einheit T4-DNA-Ligase (BRL, Maryland, USA) zugesetzt und dann das Gemisch bei 15 °C 16 Stunden lang inkubiert. Während des Ligierens treten im Reaktionsgemisch diejenigen monomeren DNA-Moleküle in Erscheinung, die die geeignet grossen, in Bruchstücken das ganze Genom enthaltenden DNA-Fragmente von Medicago sativa enthalten. Diese werden auf die im Beispiel 4b) beschriebene Weise in vitro verpackt, wobei reife Phagen entstehen. Dem die reifen Phagenpartikel enthaltenden Gemisch werden 975 $\mu$l SM-Puffer zugesetzt. Das Gemisch wird zu je 100 $\mu$l auf E. coli NM526 (NCAIM B/P/1006) titriert. Der zum Titrieren (Feststellen der Phagenanzahl) verwendete Stamm wurde auf TAA-Nährboden gezüchtet.

Die Züchtung des Wirtsorganismus und die Titration werden gemäß der Gebrauchsanweisung zu dem DNA-Kit AO1-0215 vorgenommen. Nach 12 Stunden Züchtung sind pro Platte 50 000 Plaques zu finden. Bei der Kontrollklonierung (ohne die DNA von Medicago sativa) zeigt sich kein einziger Plaque. Das bedeutet, dass eine das Genom von Medicago sativa wenigstens einmal repräsentierende Genbank entstanden ist.

Ein weiterer Beweis für die Existenz der Genbank ist der Umstand, dass in jedem von 11 untersuchten unabhängigen Plaques rekombinante DNA gefunden wurde (s. Fig. 15). Dazu wurde E. coli K-1400 (NCAIM B/P/357) mit von den 11 unabhängigen Plaques stammenden Phagen infiziert. Die Populationen wurden bei 28 °C vermehrt, wobei sich die rekombinanten Phasmide in Form von Plasmiden in den Zellen vervielfältigten. Die 11 unabhängigen Populationen wurden wie bei der Ausgangsmaterialherstellung des Beispieles 1, Abschnitt I a) beschrieben gezüchtet, die Plasmid-DNA wurde wie bei der Ausgangsmaterialherstellung des Beispieles 1, Abschnitt I b) beschrieben isoliert. Die gereinigten DNA-s werden wie in Beispiel 1 beschrieben mit EcoRI geschnitten und die entstandenen Fragmente gemäss Beispiel 2 durch Elektrophorese an Agarosegel analysiert. Das Ergebnis der Gelelektrophorese wird in Fig. 15 gezeigt.

Die in diesem Beispiel als Ausgangsmaterial verwendete gereinigte Gesamt-DNA von Medicago sativa ist wie folgt beschrieben hergestellt worden.

Herstellung der Gesamt-DNA von Medicago sativa (Luzerne)

20 g Samen von Medicago sativa werden 7 Tage lang gekeimt. Die Keimpflanzen werden gesammelt und in einer Menge von 50 g in flüssigem Stickstoff gefroren und dann im Mörser zu Pulver zerrieben. Die Masse wird mit 150 ml NESP-Puffer versetzt. Die Suspension wird bei 65 °C eine Stunde lang inkubiert und dann bei 20 °C in einem Rotor der Drehzahl 8000 min$^{-1}$ mit einer Zentrifuge Sorvall RC5 zentrifugiert. Der klare Überstand wird mit einem Gemisch von Phenol und Chloroform im Volumverhältnis von 1 : 1 vorsichtig vermischt. Dann werden die beiden Phasen durch Zentrifugieren (10 000 min$^{-1}$, 10 Minuten) voneinander getrennt. Die wäßrige Phase wird abgetrennt und dreimal mit Äther extrahiert. Nach Zugabe von 200 ml 96 gew.-%-igem Äthanol wird die ausgefallene DNA durch Zentrifugieren (Sorvall RC5, 10 000 min$^{-1}$, 5 Minuten) isoliert. Nach Abtrennen des klaren Überstandes wird die DNA im Exsikkator getrocknet. Die auf diese Weise isolierte DNA wird wie bei der Ausgangsmaterialherstellung des Beispieles 1, Abschnitt I b) beschrieben in Gegenwart von Cäsiumchlorid und Äthidiumbromid durch Ultrazentrifugieren gereinigt. Die Gesamt-DNA von Medicago sativa kann in Form eines einzigen fluoreszierenden Streifens abgetrennt werden.

Beispiel 7

Herstellung der erfindungsgemäßen rekombinanten Phasmide pGYOKI9 und pGYOKI10

12,5 $\mu$l der wie bei der unten folgenden Ausgangsmaterialherstellung beschrieben erhaltenen gereinigten DNA-Lösung des Plasmids pGYOKI1/2 werden zu 12,5 $\mu$l B-Puffer gegeben.

Das Gemisch wird mit 5 Einheiten des Restriktionsenzyms BclI (BRL, Maryland, USA) versetzt und dann eine Stunde lang bei 50 °C inkubiert.

12,5 $\mu$l der wie bei der Ausgangsmaterialherstellung des Beispieles 1, Abschnitt II b) beschrieben gereinigten EMBL4-DNA werden zu 12,5 $\mu$l B-Puffer gegeben. Das Gemisch wird mit 5 Einheiten BamHI versetzt und dann eine Stunde lang bei 37 °C inkubiert.

Das Restriktionsenzym BclI schneidet die DNA bei der Sequenz TGATCA, BamHI bei der Sequenz GGATCC. Es entstehen aus vier Basenpaaren bestehende überhängende Enden, die einander komplementär sind und deshalb in allen Kombinationen miteinander in Verbindung treten können.

Beide Reaktionsgemische werden wie bei der Ausgangsmaterialherstellung des Beispieles 4, Abschnitt b) beschrieben mit Phenol versetzt, die DNA wird mit Äthanol ausgefällt und in je 10 $\mu$l TE-Puffer gelöst. Die beiden Lösungen werden vereinigt. Zu der die linearen DNA-Moleküle enthaltenden Lösung werden 5 $\mu$l L-Lösung und eine Einheit T4-DNA-Ligase gegeben. Das Gemisch wird bei 15 °C 16 Stunden lang inkubiert. Im Reaktionsgemisch erscheinen die rekombinanten, erfindungsgemäßen Phasmide pGYOKI9 uns

EP 0 308 883 B1

pGYOKI10. Ihre Herstellung ist schematisch in Fig. 9 wiedergegeben, ihre Restriktions- und Funktionskarte zeigt Fig. 12.

Die in diesem Beispiel als Ausgangsmaterial verwendete gereinigte DNA-Lösung des Plasmides pGYOKI1/2 ist wie folgt bschrieben hergestellt worden.

a) Isolierung des Plasmids pGYOKI1

Der Stamm E. coli pGYOKI1 (NCAIM B/P/1008; hinterlegt am 4. Juni 1987) wird wie bei der Ausgangsmaterialherstellung des Beispieles 1, Abschnitt I a) beschrieben aufrechterhalten und gezüchtet. Das Plasmid pGYOKI1 wird wie bei der Ausgangsmaterialherstellung des Beispieles 1, Abschnitt I b) beschrieben isoliert. Die Restriktions- und Funktionskarte des bifunktionellen Plasmids ist in Fig. 10 dargestellt (s. auch ungarische Patentanmeldung Nr. 2468/83).

b) Herstellung des rekombinanten Plasmids pGYOKI1/2

10 $\mu$l des gemäß Abschnitt a) gereinigten Plasmids pGYOKI1 werden zu 10 $\mu$l B-Puffer gegeben und dann mit 2 Einheiten des Restriktionsendonuclease XbaI (BRL, Maryland, USA) versetzt. Das Gemisch wird bei 37°C eine Stunde lang inkubiert. Dann wird wie bei der Herstellung des Ausgangsmateriales des Beispieles 4, Abschnitt b) beschrieben das Gemisch aus Phenol und Chloroform zugesetzt, und die DNA wird mit Äthanol ausgefällt. Die gefällte DNA wird in 10 $\mu$l TE-Lösung aufgelöst.

Das Restriktionsenzym XbaI schneidet die DNA von pGYOKI1 an zwei Stellen, in der Sequenz TCTAGA. Es bilden sich an beiden DNA aus vier Basen aufgebaute kohäsive Enden, die einander komplementär sind und deshalb in jeder Kombination miteinander in Verbindung treten können.

Die in 10 $\mu$l Volumen vorliegenden, mit XbaI geschnittenen DNA-Fragmente werden auf die im Beispiel 1 beschriebene Weise in Gegenwart von einer Einheit T4-DNA-Ligase und 2,5 $\mu$l L-Puffer ligiert. Im Reaktionsgemisch erscheinen die rekombinanten Plasmide pGYOKI1/2, die aus dem grösseren Fragment des Plasmids pGYOKI1 durch Selbstligieren entstanden sind.

Die Herstellung des Plasmids pGYOKI1/2 ist schematisch in Fig. 10 dargestellt, seine Restriktions- und Funktionskarte zeigt Fig. 11.

c) Herstellung von E. coli pGYOKI1/2 (NCAIM B/P/1009; hinterlegt am 4. Juni 1987)

Mit 10 $\mu$l des gemäß Abschnitt b) erhaltenen, rekombinante Plasmide pGYOKI1/2 enthaltenden Reaktionsgemisches werden E. coli dam-minus-Zellen (NCAIM B/P/1010; hinterlegt am 4. Juni 1987) transformiert. In den dam⁻-Zellen ist kein die Methylierung von DNA kodierendes Gen vorhanden, daher wird die DNA an der Sequenz GATC nicht methyliert. Dies hat zur Folge, daß zum Beispiel die Restriktionsendonuclease BclI die aus derartigen Zellen isolierte DNA schneidet, während sie die methylierte DNA nicht beschädigt. Die Transformierung wird wie in Beispiel 2 beschrieben vorgenommen, wobei jedoch der TA-Nährboden mit E. coli dam⁻ beimpft wird.

Die isolierten transformierten Zellen werden auf einem pro $\mu$l 50 $\mu$g Chloramphenicol enthaltenden TAA-Nährboden vereinzelt. Die Kulturen werden bei 28 °C 24 Stunden lang inkubiert. Die gegen Chloramphenicol resistenten Kolonien werden abgetrennt (Transformationsfrequenz: 10 000 Transformanten/1 $\mu$g DNA) wie bei der Ausgangsmaterialherstellung des Beispieles 1, Abschnitt I a) beschrieben gezüchtet und dann wird wie bei der Ausgangsmaterialherstellung des Beispieles 1, Abschnitt I b) beschrieben die Plasmid-DNA aus ihnen isoliert.

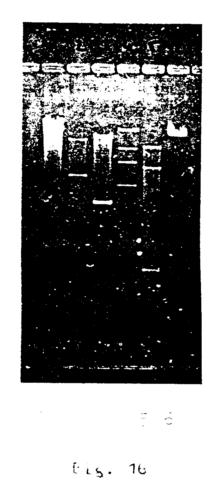

Das isolierte Plasmid pGYOKI1/2 wird mit Endonucleasen auf die im Beispiel 1 beschriebene Weise analysiert. Als Restriktionsenzyme werden EcoRI, BamHI und HindIII (BRL, Maryland, USA) verwendet. Zu je 10 $\mu$l der mit Enzym behandelten Proben werden je 10 $\mu$l D-Puffer gegeben, und die entstandenen DNA-Fragmente werden an Agarosegel durch Gelelektrophorese auf die in Beispiel 2 beschriebene Weise voneinander getrennt. Das Ergebnis der Restriktionsanalyse ist in Fig. 16 dargestellt. Die auf Grund des Ergebnisses der Analyse gezeichnete Restriktions- und Funktionskarte zeigt Fig. 11.

Der das rekombinante Plasmid pGYOKI1/2 enthaltende Stamm E. coli wurde als E. coli pGYOKI1/2 bezeichnet und unter der Nummer NCAIM B/P/1009 deponiert.

d) Isolierung des rekombinanten Plasmids pGYOKI1/2 aus den Zellen von E. coli pGYOKI1/2

Der Stamm E. coli pGYOKI1/2 (NCAIM B/P/1009) wird wie bei der Ausgangsmaterialherstellung des Beispieles 1, Abschnitt I a) beschrieben gelagert und gezüchtet. Das Plasmid wird wie bei der Ausgangs-

21

EP 0 308 883 B1

materialherstellung des Beispieles 1, Abschnitt I b) beschrieben isoliert.

Beispiel 8

Herstellung von E. coli pGYOKI9 (NCAIM B/P/1011; hinterlegt am 4. Juni 1987) und E. coli pGYOKI10 (NCAIM B/P/1012; hinterlegt am 4. Juni 1987)

5 $\mu$l des die rekombinanten erfindungsgemäßen Phasmide pGYOKI9 und pGYOKI10 enthaltenden Reaktionsgemisches von Beispiel 7 werden auf die im Beispiel 4 b) beschriebene Weise der Assemblierung unterzogen.

Mit den dabei erhaltenen reifen Phagenpartikeln, die die neuartigen Phasmide in Phagenform enthalten, werden Zellen des Stammes E. coli K-1400 (NCAIM B/P/357) infiziert. Die infizierten Zellen, die die erfindungsgemäßen Phasmide nun in Plasmidform enthalten, werden auf 50 $\mu$g/ml Chloramphenicol enthaltendem festem TAA-Nährboden vereinzelt. Die Kulturen werden bei 28 °C 24 Stunden lang inkubiert. Die CmR-Kolonien (chloramphenicol-resistent) werden abgetrennt (Assemblierungshäufigkeit in vitro: 10 000 Transformanten/ 1 $\mu$g DNA), wie bei der Ausgangsmaterialherstellung des Beispieles 1, Abschnitt I a) beschrieben gezüchtet und die Plasmid-DNA wird wie bei der Ausgangsmaterialherstellung des Beispieles 1, Abschnitt I b) beschrieben isoliert. Die isolierten Phasmide pGYOKI9 und pGYOKI10 werden wie in Beispiel 1 beschrieben mit den Restriktionsenzymen EcoRI, BamHI und HindIII (BRL, Maryland, USA) behandelt. Zu je 10 $\mu$l der mit Enzym behandelten Proben werden je 10 $\mu$l D-Puffer gegeben, und die Fragmente werden auf die im Beispiel 2 beschriebene Weise durch Gelelektrophorese an Agarosegel voneinander getrennt.

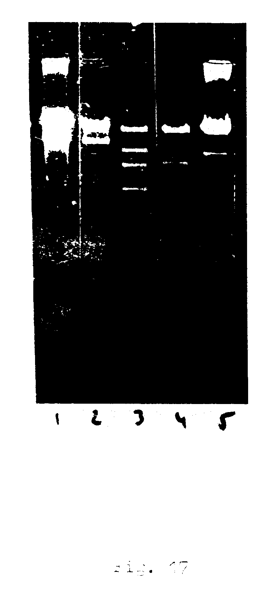

Das Ergebnis der Restriktionsanalyse zeigt Fig. 17,und in Fig. 12 ist die auf Grund der Analyse konstruierte Restriktions- und Funktionskarte dargestellt.

Der das erfindungsgemäße Phasmid pGYOKI9 enthaltende Stamm E. coli wurde als E. coli pGYOKI9 bezeichnet und unter der Nummer NCAIM B/P/1011 hinterlegt. Der hinterlegte Stamm E. coli pGYOKI10 erhielt die Nummer NCAIM B/P/1012.

Beispiel 9

Isolierung des rekombinanten erfindungsgemäßen Phasmids pGYOKI9 aus E. coli pGYOKI9 (NCAIM B/P/1011) in Form von Phagen

Der Stamm E. coli pGOKI9 (NCAIM B/P/1011) wird wie bei der Ausgangsmaterialherstellung des Beispieles 1, Abschnitt I a) beschrieben gelagert und gezüchtet. Zur Reinigung des Phasmids pGYOKI9 in Form von Phagen wird die Kultur bei 37°C auf E. coli NM526 (NCAIM B/P/1006) gemäß Beispiel 6 titriert. Bei der angegebenen Temperatur wird das neue Phasmid aus den Zellen von E. coli pGYOKI9 (NCAIM B/P/1011) in Phagenform frei und vermehrt sich in dem Stamm E. coli NM526 (NCAIM B/P/1006). Der von E. coli NM526 (NCAIM B/P/1006) als Wirtszelle erhaltene Phage wird wie bei der Ausgangsmaterialherstellung des Beispieles 1, Abschnitt II a) beschrieben vermehrt, und die Phagen-DNA wie bei der Ausgangsmaterialherstellung des Beispieles 1, Abschnitt II b) beschrieben isoliert.

Beispiel 10

Herstellung einer Genbank von Streptomyces tendae mit dem neuen Phasmid pGYOKI9

12,5 $\mu$l der die gemäß Beispiel 9 erhaltene gereinigte Phasmidphagen-DNA enthaltenden Lösung werden zu 12,5 $\mu$l B-Puffer gegeben. Des Gemisch wird mit 5 Einheiten des Restriktionsenzyms BamHI (BRL, Maryland, USA) versetzt und dann bei 37°C eine Stunde lang inkubiert. Nach dem Inkubieren wird die Probe auf die im Beispiel 4 a) beschriebene Weise mit Phosphatase behandelt und dann wie bei der Herstellung des Ausgangsmateriales des Beispieles 4, Abschnitt b) beschrieben mit Phenol versetzt. Die DNA wird mit Äthanol ausgefällt und in 20 $\mu$l TE-Lösung gelöst.

125 $\mu$l der wie bei der unten folgenden Ausgangsmaterialherstellung erhaltenen gereinigten DNA von Streptomyces tendea werden zu 125 $\mu$l B-Puffer gegeben. Das Gemisch wird mit einer Einheit der Restriktionsendonuclease Sau3AI versetzt und bei 37°C 10 Minuten lang inkubiert. Das Gemisch wird wie bei der Herstellung des Ausgangsmateriales des Beispieles 4, Abschnitt b) beschrieben mit Phenol behandelt und die DNA mit Äthanol ausgefällt. Die DNA wird in 100 $\mu$l TE-Lösung gelöst.

22

Durch das Enzym Sau3AI wird die DNA partiell gespalten, es entstehen Fragmente unterschiedlicher Grösse. Da in den Vektor Fragmente von 5-9 kb optimal eingebaut werden können, wird zur Isolierung dieser Fragmente die partiell gespaltene DNA an einem Saccharosegradienten wie in Beispiel 4 a) beschrieben aufgetrennt. Nach Beendigung des Zentrifugierens werden Fraktionen zu je 200 $\mu$l genommen und durch Gel-Elektrophorese an Agarose die Grösse der in den Fraktionen befindlichen DNA-Stücke bestimmt. Die geeigneten Fraktionen (5-9 kb) werden vereinigt, die DNA wird mit Äthanol ausgefällt und in 10 $\mu$l TE-Lösung aufgelöst.

Das Restriktionsenzym BamHI schneidet die DNA in der Sequenz GGATCC, das Restriktionsenzym Sau3AI an der Sequenz GATC. An beiden DNA entstehen aus vier Basenpaaren bestehende kohäsive Enden, die einander komplementär sind und deshalb miteinander in jeder Kombination in Verbindung treten können.

Das mit BamHI geschnittene und mit Phosphatase behandelte Phasmid pGYOKI9 wird mit der durch Sau3AI partiell gespaltenen DNA von Streptomyces tendea ligiert. Je 10 $\mu$l der beiden Lösungen werden miteinander vereinigt und mit 5 $\mu$l LP-Mischung versetzt. Um die linearen DNA-Moleküle miteinander zu verbinden, wird eine Einheit T4-DNA-Ligase zugesetzt (BRL, Maryland, USA) und dann bei 15 °C 16 Stunden lang inkubiert. Während des Ligierens erscheinen im Reaktionsgemisch diejenigen monomeren DNA-Moleküle, die die geeignet grossen, in ihrer Gesamtheit das ganze Genom von Streptomyces tendae umfassenden DNA-Fragmente enthalten.

Diese Moleküle werden auf die im Beispiel 4 b) beschriebene Weise in vitro zu kompletten Phagen verpackt. Zu dem die reifen Phagen enthaltenden Gemisch werden 975 $\mu$l SM-Puffer gegeben. Das Gemisch wird (jeweils 100 $\mu$l) auf TAA-Platten an E. coli NM526 (NCAIM B/P/1006) titriert. Die Züchtung des Wirtsorganismus und das Titrieren werden auf die in der Gebrauchsanweisung zu dem DNA-Kit A01-0215 für die Assemblierung angegebene Weise vorgenommen (s. oben). Nach 12 Stunden Wachstum sind auf jeder Platte 40 000 Plaques zu finden. Das bedeutet, dass die hergestellte Genbank das Genom von Streptomyces tendae mehrfach repräsentiert. Bei einer Kontrollklonierung (Ligieren ohne die DNA von Streptomyces tendae) entstand kein einziger Plaque.

Die in diesem Beispiel als Ausgangsmaterial verwendete gereinigte DNA von Streptomyces tendae ist wie folgt beschrieben hergestellt worden.

a) Herstellung der Gesamt-DNA von Streptomyces tendae

Der Mikroorganismus Streptomyces tendae (NCAIM B/P/274; hinterlegt am 1. Juni 1986) wird auf dem agarhaltigen Nährboden GVI/b aufrechterhalten. Schrägagar dieser Zusammensetzung wird mit den Sporen von Streptomyces tendae (NCAIM B/P/274) beimpft und bei 28°C 6 Tage lang inkubiert. Mit der Kultur werden 100 ml BI-Nährboden, der in einem Erlenmeyerkolben von 500 ml Volumen bei 121°C 20 Minuten lang sterilisiert wurde, beimpft.

Die Kulturen werden bei 28 °C 48 Stunden lang auf einer entlang eines Kreises von 2,6 cm pro Minute 260 Umdrehungen ausführenden Schüttelmaschine inkubiert. Mit 1 ml der entwickelten und kontrollierten Kultur werden 100 ml BI-Nährboden beimpft, der 0,5 Gew.-% Glycin enthält. Die Kultur wird 48 Stunden lang gezüchtet.

b) Isolierung der Gesamt-DNA von Streptomyces tendea

Aus der gemäß Abschnitt a) hergestellten Kultur wird die Gesamt-DNA wie bei der Herstellung des Ausgangsmaterials des Beispieles 4, Abschnitt b) beschrieben isoliert und dann wie bei der Ausgangsmaterialherstellung des Beispieles 1, Abschnitt I b) beschrieben in Gegenwart von Cäsiumchlorid und Äthidiumbromid durch Ultrazentrifugieren gereinigt. Die reine Gesamt-DNA kann in Form eines einzigen fluoreszierenden Streifens isoliert werden.

Legende zu den Fig. 13-17

Fig. 13: : Restriktionsanalyse der die DNA von Streptomyces tenebrarius enthaltenden rekombinanten Klone pGY95
Bande 1-12 : die nach Schneiden der aus den Klonen gewonnenen DNA mit EcoRI erhaltenen Fragmente
Bande 13 : Die nach Schneiden der DNA des Lambdaphagen mit Hind III erhaltenen Fragmente (Molmassemarkierung
Fig. 14: : Restriktionsanalyse der DNA von pGY95 und pGY97

Bande 1-3 : pGY95, geschnitten mit BamHI, EcoRI und HindIII

Bande 5-7 : geschnitten mit BamHI, EcoRI und HindIII

Bande 4 : Lambdaphagen-DNA, geschnitten mit HindIII

Fig. 15: : Restriktionsanalyse der die DNA von Medicago sativa (Luzerne) enthaltenden rekombinanten Klone pGY97

Bande 1-11: aus den Klonen isolierte DNA, geschnitten mit EcoRI

Bande 12 : Lambdaphagen-DNA, geschnitten mit HindIII

Fig. 16: Restriktionsanalyse des Plasmids pGYOKI1/2

Die Fragmente von pGYOKI1/2

Bande 1: nach Schneiden mit BamHI

Bande 2: nach Schneiden mit BclI

Bande 3: nach Schneiden mit EcoRI

Bande 4: Lambdaphagen-DNA, geschnitten mit HindIII

Bande 5: nach Schneiden mit HindIII

Bande 6: nach Schneiden mit PstI

Fig. 17: Restriktionsanalyse der DNA von pGYOKI9 und pGYOKI10

Bande 1: pGYOKI9, geschnitten mit EcoRI

Bande 2: pGYOKI10,geschnitten mit EcoRI

Bande 3: Lambdaphagen-DNA, geschnitten mit HindIII (Marker)

Bande 4: pGYOKI9, geschnitten mit HindIII

Bande 5: pGYOKI10, geschnitten mit HindIII

## Patentansprüche
### Patentansprüche für folgende Vertragsstaaten : AT, BE, CH, DE, FR, GB, IT, LI, NL, SE

1. Verfahren zum Klonieren von DNA-Sequenzen beziehungsweise zur Herstellung von Genbanken, dadurch gekennzeichnet, daß ein linearer Vektor verwendet wird, welcher zunächst mit Restriktionsendonuklease behandelt wird, und dem anschließend mit Phosphatase die Phosphatgruppen sowohl von den freien cos 5'-Enden als auch von den durch die Behandlung mit Restriktionsendonuklease erzeugten Restriktions-5'-Enden entfernt werden, zu diesen zur Ligation untereinander unfähigen Fragmenten werden dann die das zu klonierende Gen enthaltenden, an ihren Enden Phosphatgruppen aufweisenden DNA-Fragmente, zugegeben und das Gemisch mit Ligase ligiert, und die erhaltene monomere DNA in vitro verpackt und dann die Phagen vermehrt.

2. Verfahren nach Anspruch 1, dadurch gekennzeichnet, daß man das Klonieren mit dem in Fig. 7 gezeigten Phasmid pGY97 als DNA-klonierendem Vektor durchführt.

3. Verfahren nach Anspruch 1, dadurch gekennzeichnet, daß man das Klonieren mit dem in Fig. 12 gezeigten Phasmid pGYOKI9 als DNA-klonierenden Vektor durchführt.

4. Verfahren nach Anspruch 1, dadurch gekennzeichnet, daß man das Klonieren mit dem in Fig. 12 gezeigten Phasmid pGYOKI10 als DNA-klonierenden Vektor durchführt.

5. Phasmide pGYOKI9 und pGYOKI10, gekennzeichnet durch die aus Fig. 12 ersichtlichen Restriktions- und Funktionskarten, je ein Chloramphenicol-, Neomycin- und Thiostrepton-Resistenzgen und eine Größe von 41 kb.

6. Mikroorganismusstamm Escherichia coli pGYOKI9 (NCAIM B/P/1011), enthaltend das Phasmid pGYOKI9 nach Anspruch 5.

7. Mikroorganismusstamm Escherichia coli pGYOKI10 (NCAIM B/P/1012), enthaltend das Phasmid pGYOKI10 nach Anspruch 5.

### Patentansprüche für folgenden Vertragsstaat : ES

1. Verfahren zum Klonieren von DNA-Sequenzen beziehungsweise zur Herstellung von Genbanken, dadurch gekennzeichnet, daß ein linearer Vektor verwendet wird, welcher zunächst mit Restriktionsendonuklease behandelt wird, und dem anschließend mit Phosphatase die Phosphatgruppen sowohl von den freien cos 5'-Enden als auch von den durch die Behandlung mit Restriktionsendonuklease erzeugten Restriktions-5'-Enden entfernt werden, zu diesen zur Ligation untereinander unfähigen Fragmenten werden dann die das zu klonierende Gen enthaltenden, an ihren Enden Phosphatgruppen aufweisenden DNA-Fragmente, zugegeben und das Gemisch mit Ligase ligiert, und die erhaltene monomere DNA in vitro verpackt und dann die Phagen vermehrt.

2. Verfahren nach Anspruch 1, dadurch gekennzeichnet, daß man das Klonieren mit den in Fig. 7 gezeigten Phasmid pGY97 als DNA-klonierendem Vektor durchführt.

3. Verfahren nach Anspruch 1, dadurch gekennzeichnet, daß man das Klonieren mit dem in Fig. 12 gezeigten Phasmid pGYOKI9 als DNA-klonierendem Vektor durchführt.

4. Verfahren nach Anspruch 1, dadurch gekennzeichnet, daß man das Klonieren mit dem in Fig. 12 gezeigten Phasmid pGYOKI10 als DNA-klonierendem Vektor durchführt.

**Claims**
**Claims for the following Contracting States : AT, BE, CH, DE, FR, GB, IT, LI, NL, SE**

1. A method of cloning DNA sequences and of producing gene banks, characterised in that a linear vector is used which is first treated with restriction endonuclease and from which the phosphate groups are then removed with phosphatase both from the free cos 5'-ends and from the restriction 5'-ends produced by the treatment with restriction endonuclease, there are then added to those fragments, which are not capable of ligation with one another, the DNA fragments containing the gene to be cloned and having phosphate groups at their ends, and the mixture is ligated with ligase, and the resulting monomeric DNA is packaged in vitro and then the phages are replicated.

2. Method according to claim 1, characterised in that the cloning is carried out with the phasmid pGY97 shown in Fig. 7 as the DNA-cloning vector.

3. Method according to claim 1, characterised in that the cloning is carried out with the phasmid pGYOKI9 shown in Fig. 12 as the DNA-cloning vector.

4. Method according to claim 1, characterised in that the cloning is carried out with the phasmid pGYOKI10 shown in Fig. 12 as the DNA-cloning vector.

5. Phasmids pGYOKI9 and pGYOKI10, characterised by the restriction and function maps that can be seen in Fig. 12, by a chloramphenicol-, a neomycin- and a thiostreptone-resistance gene and a size of 41 kb.

6. Microorganism strain Escherichia coli pGYOKI9 (NCAIM B/P/1011) containing the phasmid pGYOKI9 according to claim 5.

7. Microorganism strain Escherichia coli pGYOKI10 (NCAIM B/P/1012) containing the phasmid pGYOKI10 according to claim 5.

**Claims for the following Contracting State : ES**

1. A method of cloning DNA sequences and of producing gene banks, characterised in that a linear vector is used which is first treated with restriction endonuclease and from which the phosphate groups are then removed with phosphatase both from the free cos 5'-ends and from the restriction 5'-ends produced by the treatment with restriction endonuclease, there are then added to those fragments, which are not capable of ligation with one another, the DNA fragments containing the gene to be cloned and having phosphate groups at their ends, and the mixture is ligated with ligase, and the resulting monomeric DNA is packaged in vitro and then the phages are replicated.

2. Method according to claim 1, characterised in that the cloning is carried out with the phasmid pGY97 shown in Fig. 7 as the DNA-cloning vector.

**3.** Method according to claim 1, characterised in that the cloning is carried out with the phasmid pGYOKI9 shown in Fig. 12 as the DNA-cloning vector.

**4.** Method according to claim 1, characterised in that the cloning is carried out with the phasmid pGYOKI10 shown in Fig. 12 as the DNA-cloning vector.

**Revendications**

**Revendications pour les Etats contractants suivants : AT, BE, CH, DE, FR, GB, IT, LI, NL, SE**

**1.** Procédé de clonage de séquences d'ADN et de préparation de banques de gènes, caractérisé en ce qu'on utilise un vecteur linéaire qu'on traite d'abord avec une endonucléase de restriction et dont on enlève ensuite, avec une phosphatase, les groupes phosphates des extrémités libres cos 5' ainsi que des extrémités de restriction 5' produites par le traitement avec l'endonucléase de restriction, ensuite on ajoute à ces fragments, inaptes à être ligaturés entre eux, les fragments d'ADN contenant le gène à cloner et qui présentent les groupes phosphates à leurs extrémités, on soumet le mélange à une ligation avec une ligase, l'ADN monomère obtenu est encapsidé in vitro et ensuite, les phages sont multipliés.

**2.** Procédé selon la revendication 1, caractérisé en ce qu'on effectue le clonage avec le phasmide pGY97 qui est représenté sur la figure 7, comme vecteur de clonage de l'ADN.

**3.** Procédé selon la revendication 1, caractérisé en ce qu'on effectue le clonage avec le phasmide pGYOKI9 qui est représenté sur la figure 12, comme vecteur de clonage de l'ADN.

**4.** Procédé selon la revendication 1, caractérisé en ce qu'on effectue le clonage avec le phasmide pGYOKI10 qui est représenté sur la figure 12, comme vecteur de clonage de l'ADN.

**5.** Phasmides pGYOKI9 et pGYOKI10, caractérisés par les cartes de restriction et de fonction représentées sur la figure 12, ayant chacun un gène de résistance au chloramphénicol, à la néomycine et à la thiostreptone, et une dimension de 41 kb.

**6.** Souche de micro-organisme Escherichia coli pGYOKI9 (NCAIM B/P/1011) contenant le phasmide pGYOKI9 selon la revendication 5.

**7.** Souche de micro-organisme Escherichia coli pGYOKI10 (NCAIM B/P/1012) contenant le phasmide pGYOKI10 selon la revendication 5.

**Revendications pour l'Etat contractant suivant : ES**

**1.** Procédé de clonage de séquences d'ADN et de préparation de banques de gènes, caractérisé en ce qu'on utilise un vecteur linéaire qu'on traite d'abord avec une endonucléase de restriction et dont on enlève ensuite, avec une phosphatase, les groupes phosphates des extrémités libres cos 5' ainsi que des extrémités de restriction 5' produites par le traitement avec l'endonucléase de restriction, ensuite on ajoute à ces fragments, inaptes à être ligaturés entre eux, les fragments d'ADN contenant le gène à cloner et qui présentent les groupes phosphates à leurs extrémités, on soumet le mélange à une ligation avec une ligase, l'ADN monomère obtenu est encapsidé in vitro et ensuite, les phages sont multipliés.

**2.** Procédé selon la revendication 1, caractérisé en ce qu'on effectue le clonage avec le phasmide pGY97 qui est représenté sur la figure 7, comme vecteur de clonage de l'ADN.

**3.** Procédé selon la revendication 1, caractérisé en ce qu'on effectue le clonage avec le phasmide pGYOKI9 qui est représenté sur la figure 12, comme vecteur de clonage de l'ADN.

**4.** Procédé selon la revendication 1, caractérisé en ce qu'on effectue le clonage avec le phasmide pGYOKI10 qui est représenté sur la figure 12, comme vecteur de clonage de l'ADN.

Fig.1

Klonieren in einen Phagenvektor

Fig. 2

Klonierung in ein Cosmid

Fig. 3

Schematische Darstellung der Herstellung von pGY 95 und pGY 97

Fig. 4

Restriktions- und Funktionskarte von pGY 95

Fig. 5

Restriktions- und Funktionskarte von pGY 97

Fig.7

EP 0 308 883 B1

Klonieren in ein neuartiges Phasmid
pGYOKI 9

Fig. 8

Schematische Abbildung der Herstellung von
pGYOKI9 und pGYOKI 10

Fig. 9

# Schematische Abbildung der Herstellung von pGYOKI 1/2

— pACYC184
— pIJ41

Fig.10

EP 0 308 883 B1

Restriktions- und Funktionskarte von pGYOKI 1/2

— pACYC 184
— plJ 41

Fig. 11

36

Restriktions– und Funktionskarte von pGYOKI 9 und pGYOKI 10

Fig.12

Fig 13

1 2 3 4 5 6 7

Fig. 14

Fig. 15

fig. 16

Fig. 17